# EUROPEAN PATENT APPLICATION

(11) **EP 4 265 248 A1**
(43) Date of publication of application: **25.10.2023**
(21) Application number: 22169555.4
(22) Date of filing: 22.04.2022
(51) Int. Cl.: A61K 31/437, A61K 31/444, A61K 35/00, A61K 45/06, A61P 37/08, C07D 471/04

(54) **COMPOUNDS INDUCING PRODUCTION OF PROTEINS BY IMMUNE CELLS**

(71) Applicant: Université Paris Cité, 75006 Paris (FR); Centre National de la Recherche Scientifique, 75016 Paris (FR); Institut National de la Santé et de la Recherche Médicale, 75013 Paris (FR); Institut Curie, 75005 Paris (FR); Université Paris-Saclay, 91190 Gif-sur-Yvette (FR)
(72) Inventor: FILLATREAU, Simon, 75017 Paris (FR); MAHUTEAU-BETZER, Florence, 78470 Saint Rémy-lès-Chevreuse (FR); BEAUVINEAU, Claire, 91440 Bures-sur-Yvette (FR); BORZAKIAN, Sibyline, 75003 PARIS (FR)
(74) Representative: Cabinet Beau de Loménie

(57) **Abstract**

The present invention relates to a compound of formula (I) for its use as an inductor compound for the production of interleukin-10 (IL-10) by immune cells. The invention also relates to induced immune cells capable of producing interleukin 10. The invention also relates to the use of the induced immune cells in the prevention and/or treatment of immune-mediated diseases, in particular neurodegenerative diseases, inflammatory diseases, allergic diseases, adverse events related to transplantation, autoimmune diseases, metabolic diseases with an inflammatory component, inflammation facilitating cancer development or progression, and aging.

## Description

### FIELD OF THE INVENTION

The present invention relates to compounds inducing the production of proteins, in particular interleukin-10 (IL-10) by immune cells and its use in the prevention and/or treatment of immune-mediated diseases, in particular neurodegenerative diseases, inflammatory diseases, allergic diseases, adverse events related to transplantation, autoimmune diseases, metabolic diseases with an inflammatory component, inflammatory responses having a deleterious impact on the risk of cancer development or cancer progression, and aging.

### BACKGROUND OF THE INVENTION

Immune-mediated diseases are chronic inflammatory diseases perpetuated by antibodies and cellular immunity. The immune response damages healthy organs either inadvertently as a result of attacking foreign ingredients that have entered the body, or by attacking self tissues, a process called autoimmunity. These diseases include many forms of arthritis (e.g., rheumatoid arthritis and psoriatic arthritis), inflammatory bowel diseases (e.g., ulcerative colitis and Crohn's disease), endocrinopathies (e.g., type 1 diabetes and Graves disease), neurodegenerative diseases (e.g., multiple sclerosis, autistic spectrum disorder, Alzheimer's disease, amyotrophic lateral sclerosis (ALS), Parkinson's disease, Huntington's Disease, Guillain-Barre syndrome, myasthenia gravis, and chronic idiopathic demyelinating disease (CID)), and vascular diseases (e.g., autoimmune hearing loss, systemic vasculitis, and atherosclerosis). These diseases are common and have a major socioeconomic impact.

Currently, the primary focus of therapy is to suppress immunity and inhibit inflammation, by using immunosuppressive drugs, blockers of cytokine or cytokine receptor signaling, or by the transient depletion of leukocyte subsets. However, these approaches do not take into account the need to restore dominant immune regulation, which is often defective in these pathologies, to restore a healthy and stable immune system. A large fraction of patients treated by these drugs do not respond, or become resistant after some time, or relapse upon treatment cessation.

Thus, there is a need for new effective forms of therapy, in particular treatments that may provide a controlled, sustained therapy, offered alone or in combination with other therapies, whilst improving patient quality of life and reducing side effects related to treatment.

To prevent and/or treat immune-mediated diseases, the inventors have developed a new compound inducing production of proteins, in particular interleukin-10 (IL-10), by immune cells, enabling the generation of IL-10-producing immune cells, which can be used in adoptive cell therapy.

### SUMMARY OF THE INVENTION

The present invention provides a compound inducing production of proteins, in particular interleukin-10 (IL-10) by immune cells of formula (I): 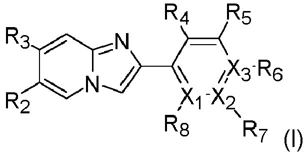 wherein:
- X₁, X₂, X₃ identical or different, are each independently selected from a nitrogen atom and a carbon atom ;
- R₂ and R₃, identical or different, are each independently selected from:
   - a hydrogen atom,
   - a halogen atom,
   - a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
   - a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a -NO₂ group, a -OCF₃ group, a -CF₃ group;
   - a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
   - a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a-NO₂ group, a -OCF₃ group, a -CF₃ group;
- R₄, R₅, R₆, R₇ and R₈, identical or different, are each independently selected from :
   - a hydrogen atom,
   - a cyano group,
   - a halogen atom, and
   - a -NHCOR_{b} group, wherein R_{b} is selected from a (C₁-C₆)cycloalkyl group, a (C₁-C₄)alkyl group, a (C₁-C₄)allyl group, a (C₁-C₄)alkoxy group, a pyridine group and a phenyl group substituted by (Z₈)_{b}, wherein : b is selected from 0, 1, 2, 3, 4 and 5, and Z₈, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
      with the proviso that
   - If X₁ is a nitrogen atom, R₈ is absent,
   - If X₂ is a nitrogen atom, R₇ is absent,
   - If X₃ is a nitrogen atom, R₆ is absent.

The present invention also provides a method for inducing immune cells characterized in that said immune cells are contacting with at least one compound of formula (I) inducing production of proteins, in particular interleukin-10.

The present invention also provides an immune cell capable of producing proteins, in particular interleukin-10.

The present invention also provides a pharmaceutical composition comprising a dose of induced immune cell, and at least one pharmaceutically acceptable carrier and its use in the prevention and/or treatment of immune-mediated diseases, in particular neurodegenerative diseases, inflammatory diseases, allergic diseases, adverse events related to transplantation, autoimmune diseases, metabolic diseases with an inflammatory component, inflammation facilitating cancer development or progression, and aging.

### DESCRIPTION OF FIGURES

**Figure 1****:** Effect of compounds 133, 161, 77 and 91 on IL-10 induction by human B cells.
**Figure 2****:** Effect of induction of human B cells with compound 133 on IL-10 production by human B cells
**Figure 3****:** Effect of induction of human B cells with compound 77 on IL-10 production by human B cells
**Figure 4****:** Effect of induction of human B cells with compound161 on IL-10 production by human B cells
**Figure 5****:** Effect of induction of human B cells with compound 91 on IL-10 production by human B cells

### DETAILLED DESCRIPTION

The present invention provides a compound inducing production of proteins, in particular interleukin-10 (IL-10) by immune cells of formula (I): 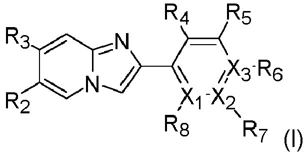 wherein:
- X₁, X₂, X₃ identical or different, are each independently selected from a nitrogen atom and a carbon atom ;
- R₂ and R₃, identical or different, are each independently selected from:
   - a hydrogen atom,
   - a halogen atom,
   - a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
   - a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a -NO₂ group, a -OCF₃ group, a -CF₃ group;
   - a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
   - a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a-NO₂ group, a -OCF₃ group, a -CF₃ group;
- R₄, R₅, R₆, R₇ and R₈, identical or different, are each independently selected from :
   - a hydrogen atom,
   - a cyano group,
   - a halogen atom, and
   - a -NHCOR_{b} group, wherein R_{b} is selected from a (C₁-C₆)cycloalkyl group, a (C₁-C₄)alkyl group, a (C₁-C₄)allyl group, a (C₁-C₄)alkoxy group, a pyridine group and a phenyl group substituted by (Z₈)_{b}, wherein : b is selected from 0, 1, 2, 3, 4 and 5, and Z₈, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
      with the proviso that
   - If X₁ is a nitrogen atom, R₈ is absent,
   - If X₂ is a nitrogen atom, R₇ is absent,
   - If X₃ is a nitrogen atom, R₆ is absent.

The inventors have shown that the compound of formula (I) is capable of inducing production of proteins, in particular interleukin 10 (IL-10) by immune cells, without increasing production of interleukin-6 (II-6). Advantageously, the compound of formula (I) enables the production of pure and stable IL-10 producing immune cells. In particular, the inventors have shown that the compound of formula (I) increases interleukin production, in particular IL-10 production, by immune cells, in particular B cells, T cells or myeloid cells, activated via i) Toll-like receptors (TLR) and IgM for B cells, ii) TLR for myeloid cells, as well as iii) CD3 and CD28 and IL-27 receptor for T cells, indicating that the IL-10 production is not restricted to any particular activation condition. Moreover, the inventors have shown that the compound of formula (I) induces a distinct molecular program in immune cell, in particular B cell, T cells or myeloid cells, with an up-regulation of transcriptional regulators known to promote IL-10 expression in these cells.

The inventors have shown that immune cells induce with a compound of formula (I) continue to express proteins, in particular IL-10 homogeneously, when subsequently washed and re-cultured in various conditions with the compound of formula (I), while the immune cells were refractory to IL-6 production.

As used herein, the term "compound inducing production of proteins" refer to a compound enables to increase proteins production by immune cell. Advantageously, a compound inducing production of proteins enables to produce immune cell cultures, wherein more than 5%, advantageously more than 10%, advantageously more than 15%, advantageously more than 20%, advantageously more than 25%, advantageously more than 30%, advantageously more than 35%, advantageously more than 40%, advantageously more than 45%, advantageously more than 50%, advantageously more than 55%, advantageously more than 60%, advantageously more than 65%, advantageously more than 70%, advantageously more than 75%, advantageously more than 80%, advantageously more than 85%, advantageously more than 90%, advantageously more than 91%, advantageously more than 92%, advantageously more than 93%, advantageously more than 94%, advantageously more than 95%, advantageously more than 96%, advantageously more than 97%, advantageously more than 98%, advantageously more than 99% of the immune cells of the culture are capable of producing proteins. Advantageously, the compound of the invention induces production of proteins, in particular of one of the following proteins: interleukin-10, PDL1, PDL2, CD200, KLRA4 and LAG-3, advantageously the production of interleukin-10.

As used herein, the term "interleukin-10", also known as human cytokine synthesis inhibitory factor (CSIF), is an anti-inflammatory cytokine. As used herein, the terms "interleukin-10" or "IL10" or "IL-10" are interchangeable.

As used herein, the term "Ct-Cz" means a carbon chain optionally containing from t to z carbon atoms in which t and z can take values from 1 to 10; for example, C₁-C₆ is a carbon chain optionally containing 1 to 6 carbon atoms.

As used herein, the term "alkyl group" refers to a straight or branched hydrocarbon chain group consisting solely of carbon and hydrogen atoms, which is saturated or unsaturated (i.e., contains one or more double and/or triple bonds). In particular, a "C₁-C₆ alkyl group" refers an alkyl group having 1 to 6 carbon atoms. By way of examples, mention may be made of methyl, ethyl, n-propyl, isopropyl, butyl, n-butyl, isobutyl, tert-butyl, pentyl, neopentyl, n-hexyl and the like. "C₁ alkyl" refers to methyl, "C₂ alkyl" refers to ethyl, "C₃ alkyl" refers to propyl and "C₄ alkyl" refers to butyl.

As used herein, the term "C₁-C₆ alkoxy group" refers to a straight or branched hydrocarbon chain group comprising the specified number of carbon atoms, advantageously between 1 and 6 carbon atoms, and an oxygen atom (-O-C). By way of examples, mention may in particular be made of a methoxy, ethoxy, propoxy, isopropoxy, butoxy, tert-butoxy, pentoxy, and hexoxy. "C₁ alkoxy" refers to methoxy, "C₂ alkoxy" refers to ethoxy, "C₃ alkoxy" refers to propoxy and"C₄ alkoxy" refers to butoxy. Further, as used herein, "OMe" refers to methoxy and "OEt" refers to ethoxy.

As used herein, the term "cycloalkyl" refers to a monovalent or multivalent saturated ring having 1 to 12 ring carbon atoms as a monocyclic, bicyclic, or tricyclic ring system, and wherein the bicyclic or tricyclic ring system may include fused ring, briged ring and spiro ring. In some embodiments, the cycloalkyl group is C₁-C₆ cycloalkyl which contains 1 to 6 ring carbon atoms. Examples of cycloalkyl group include, but are not limited to, cyclopropyl, cyclobutyl, cyclopentyl, cyclohexyl, and the like. The cycloalkyl radical is optionally substituted with one or more substituents described herein.

As used herein, the term "pyridine" refers to a six-membered heteroaromatic ring with one nitrogen atom.

As used herein, the term "phenyl" refers to an aromatic ring system including six carbon atoms (commonly referred to as benzene ring). The phenyl is optionally substituted with one or more substituents described herein.

As used herein, the term "allyl group" refers to an atomic group represented by "CH₂=CH-CH₂- or a derivative thereof (e.g. allyl chloride), as a monovalent unsaturated hydrocarbon.

As used herein, the term "cyano" refers to a group -CN or a group -alkyl-CN, wherein alkyl is as herein defined.

As used herein, the term "alkylether group" refers to any alkyl group as defined above, wherein at least one carbon-carbon bond is replaced with a carbon-oxygen bond. The carbon-oxygen bond may be on the terminal end (as in an alkoxy group) or the carbon oxygen bond may be internal (i.e., C-O-C).

As used herein, the term "hydroxyl" refers to a monovalent group of formula -OH.

As used herein, the term "halogen atom" refers to an atom selected from fluorine, chlorine, bromine, or iodin. The halogen atom may advantageously be a fluorine atom or a bromine atom.

According to the present invention, the compound of formula (I) according to the invention can be used in the form of a pharmaceutically acceptable salt and can comprise salts, hydrates and solvates prepared according to conventional methods, well known to those skilled in the art. By way of examples, mention may in particular be made of salts derived from carboxylic acids, such as carboxylates (COO⁻), but also sulfonates (-SO₃⁻), phosphonates (PO₃²⁻), quaternary ammoniums (NR₄⁺) or sulfonamines (SO₂⁻NH₃⁺). Advantageously, suitable salts include pharmaceutically or physiologically acceptable acid addition salts. Advantageously, suitable salts include acid addition salts formed with various pharmaceutically or physiologically acceptable free acids. Examples of acids may include, but are not limited to, hydrochloric acid, bromic acid, sulfuric acid, phosphoric acid, citric acid, acetic acid, lactic acid, tartaric acid, fumaric acid, formic acid, propionic acid, oxalic acid, trifluoroacetic acid, methanesulfonic acid, benzenesulfonic acid, maleic acid, benzoic acid, gluconic acid, glycolic acid, succinic acid, 4-morpholineethanesulfonic acid, camphorsulfonic acid, nitrobenzenesulfonic acid, hydroxy-O-sulfonic acid, 4-toluenesulfonic acid, ruktu knife, EMBO acid, glutamic acid, aspartic acid, etc.

Additionally, pharmaceutically acceptable metal salts can be prepared using bases. For example, alkali metal or alkaline earth metal salts can be obtained by dissolving the compound in an excess of an alkali metal hydroxide or alkaline earth metal hydroxide solution, filtering the compound salts undissolved and evaporating and drying the filtrate. Here, sodium, potassium, lithium, cesium, magnesium or calcium salts are pharmaceutically suitable metal salts, but not limited to these. In addition, silver salts corresponding to metal salts can be obtained by reacting alkali metals or alkaline earth metals with appropriate silver salts (eg nitrates). Mention may also be made of ammoniums (NH₄⁺) or amines in ammonium form such as diethylamine (EDTA), pyrrolidine, piperidine and pyridine.

In a particular embodiment, the compound inducing production of proteins, in particular interleukin-10 (IL-10), according to the invention, wherein:
- X₁, X₂ and X₃ are a carbon atom,
- R₂ and R₃ identical or different, are each independently selected from :
   - a hydrogen atom,
   - a halogen atom,
   - a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z4, identical or different, are each independently selected from a halogen atom, a hydroxyl group, a (C₁-C₆) alkoxy group, a (C₁-C₄)alkyl group, a -NH2 group, a -NO2 group, a -OCF3 group, a -CF3 group,
   - a -NHR_{c} group, wherein R_{c} is selected from : a (C₁-C₆)cycloalkyl group, a (C₁-C₄)alkyl group, a (C₁-C₄)allyl group, a (C₁₋C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a-NO₂ group, a -OCF₃ group, a -CF₃ group;
   - a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
   - a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, and a-CF₃ group;
- R₄, R₅, R₆ and R₇, identical or different, are each independently selected from a hydrogen atom, a cyano group and a halogen atom.

Advantageously, the compound inducing production of proteins, in particular interleukin-10 (IL-10) of formula (I) according to the invention is selected among 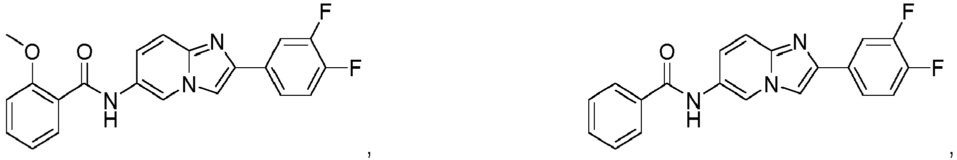 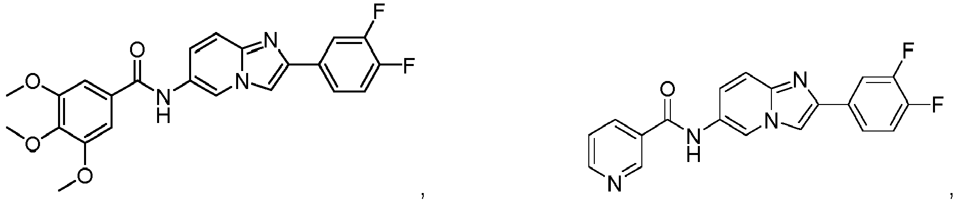 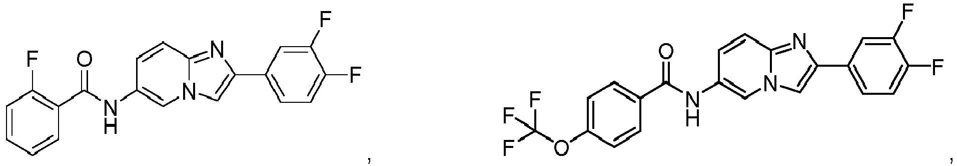 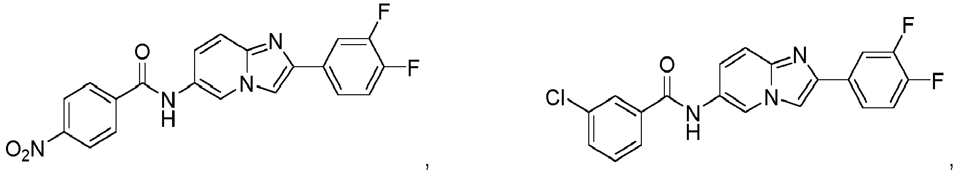 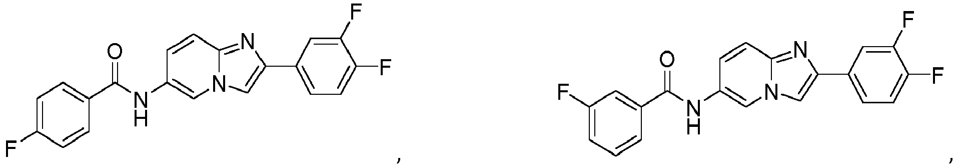 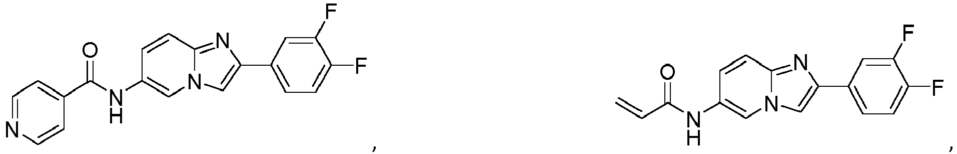 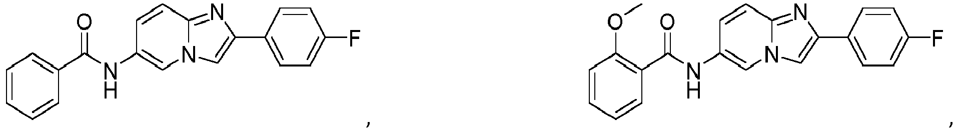 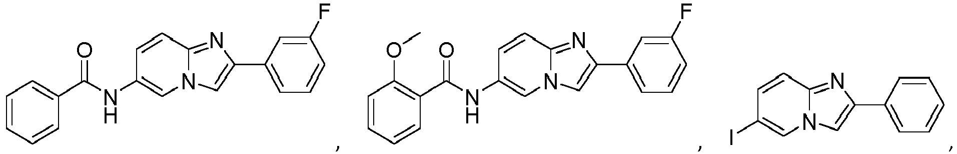 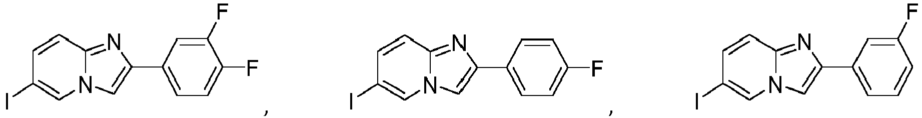 and

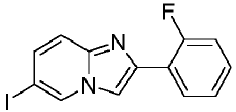

In another particular embodiment, the compound inducing production of proteins, in particular interleukin-10 (IL-10), according to the invention, wherein:
- X₁ is a nitrogen atom
- X₂, X₃, are a carbon atom,
- R₂ and R₃ identical or different, are each independently selected from :
   - a hydrogen atom,
   - a halogen atom,
   - a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
   - a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a-NO₂ group, a -OCF₃ group, a -CF₃ group;
   - a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
   - a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a -NO₂ group, a -OCF₃ group, a -CF₃ group;
- R₄, R₅, R₆ and R₈, identical or different, are each independently selected from a hydrogen atom, a cyano group and a halogen atom.

In another particular embodiment, the compound inducing production of proteins, in particular interleukin-10 (IL-10), according to the invention, wherein:
- X₂ is a nitrogen atom
- X₁, X₃, are a carbon atom,
- R₂ and R₃ identical or different, are each independently selected from :
   - a hydrogen atom,
   - a halogen atom,
   - a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
   - a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a-NO₂ group, a -OCF₃ group, a -CF₃ group;
   - a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
   - a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a -NO₂ group, a -OCF₃ group, a -CF₃ group;
- R₄, R₅, R₆ and R₈, identical or different, are each independently selected from a hydrogen atom, a cyano group and a halogen atom.

Advantageously, the compound inducing production of proteins, in particular interleukin-10 (IL-10) of formula (I) according to the invention is selected among: .

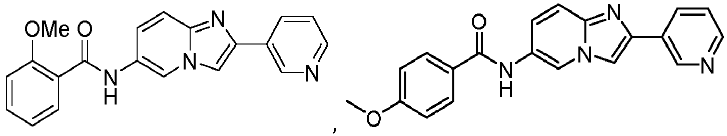

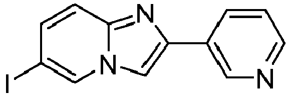

In another particular embodiment, the compound inducing production of proteins, in particular interleukin-10 (IL-10), according to the invention, wherein:
- X₃ is a nitrogen atom
- X₁ and X₂ are a carbon atom,
- R₂ and R₃ identical or different, are each independently selected from :
   - a hydrogen atom,
   - a halogen atom,
   - a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z4, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
   - a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a-NO₂ group, a -OCF₃ group, a -CF₃ group;
   - a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group; and
   - a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
- R₄, R₅, R₇ and R₈, identical or different, are each independently selected from a hydrogen atom, a cyano group and a halogen atom.

In another particular embodiment, the compound inducing production of proteins, in particular interleukin-10 (IL-10), according to the invention, wherein:
- X₂ is a nitrogen atom,
- X₁ and X₃ are a carbon atom,
- R₂ and R₃ identical or different, are each independently selected from a hydrogen atom and a halogen atom, and a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C6) alkoxy group, a (C₁-C₄)alkyl group, a -NH₂ group, a -NO₂ group, a -OCF₃ group, a -CF₃ group
- R₄, R₅, R₆ and R₈, identical or different, are each independently selected from a hydrogen atom, a cyano group and a halogen atom.

Advantageously, the compound inducing production of proteins, in particular interleukin-10 (IL-10) according to the invention, selected from:

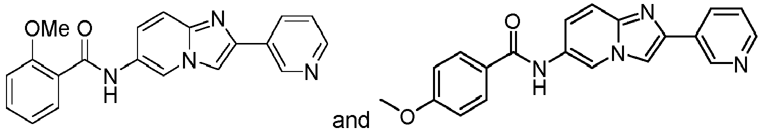

In another particular embodiment, the compound inducing production of proteins, in particular interleukin-10 (IL-10), according to the invention, wherein:
- X₂ is a nitrogen atom or a carbon atom,
- X₁ and X₃ are a carbon atom,
- R₂ and R₃ identical or different, are each independently selected from a hydrogen atom and a halogen atom,
- R₄, R₅, R₆ and R₈, identical or different, are each independently selected from a hydrogen atom, a cyano group and a halogen atom.

Advantageously, the compound inducing production of proteins, in particular interleukin-10 (IL-10) according to the invention, selected from:

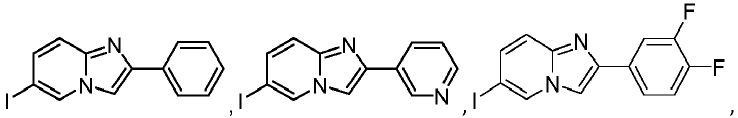

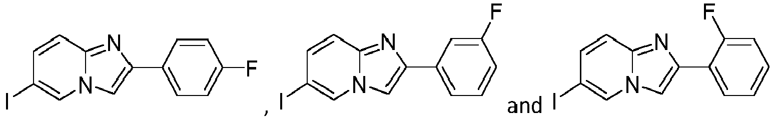

In another particular embodiment, the compound inducing production of proteins, in particular interleukin-10 (IL-10) of formula (I) according to the invention, is selected from :

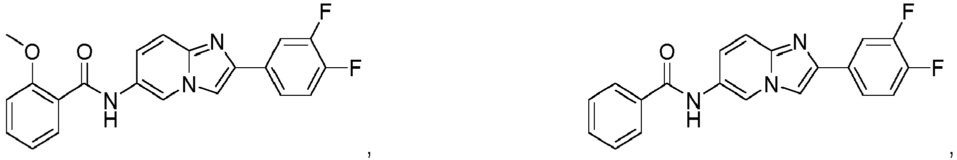

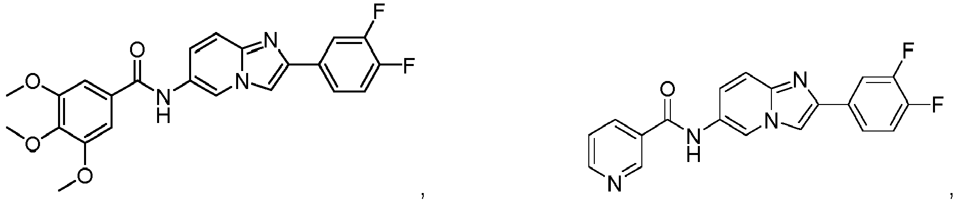

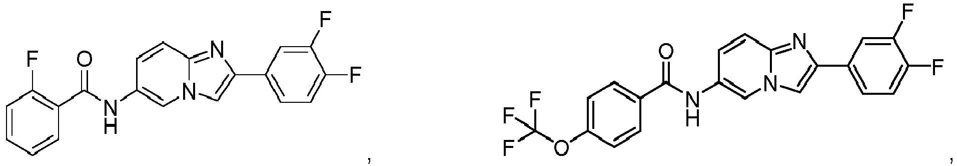

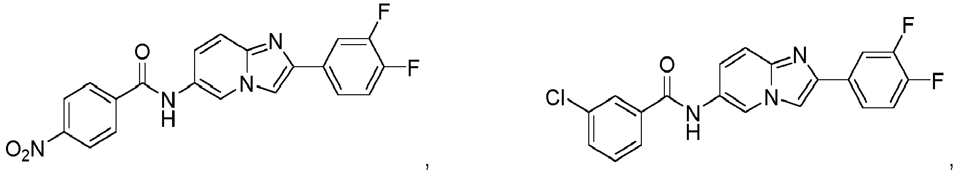

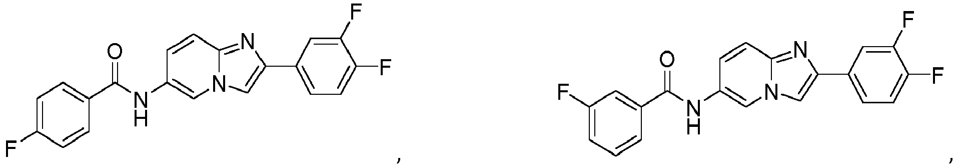

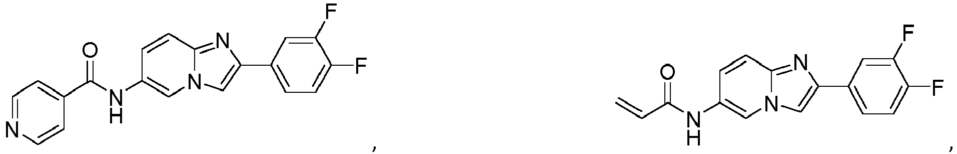

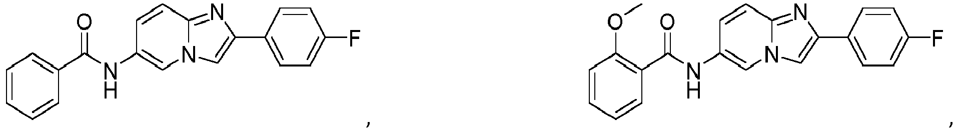

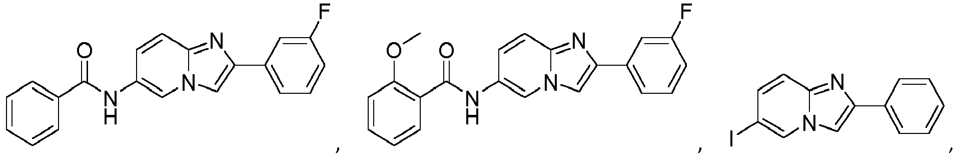

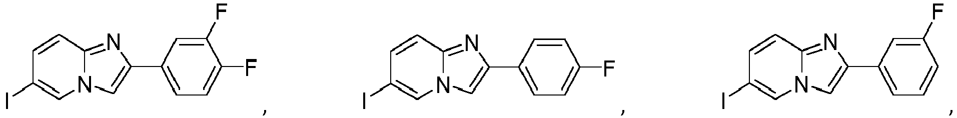

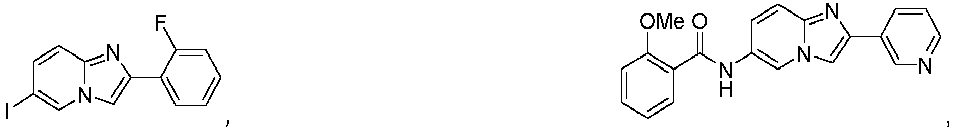

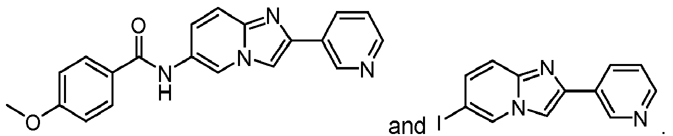

The chemical structures and some spectroscopic datas of preferred compounds of inducing production of interleukin-10 (IL-10) of formula (I) are illustrated in the following Table 1.

**Table 1 : Preferred compounds**

| **N°** | **Structure** | **Name of the compound** | **Characterization** |
|---|---|---|---|
| **212** | 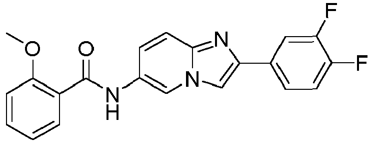 | N-(2-(3,4-difluorophenyl)imidazo[1,2-a]pyridin-6-yl)-2-methoxybenzamide | 1H NMR (300 MHz, CDCl3) δ 9.86 (s, 1H), 9.45 (s, 1H), 8.30 (dd, J = 7.8, 1.8 Hz, 1H), 7.83 (s, 1H), 7.78 (ddd, J = 11.6, 7.7, 2.1 Hz, 1H), 7.68 - 7.51 (m, 3H), 7.24 - 7.14 (m, 2H), 7.08 (d, J = 8.3 Hz, 1H), 6.95 (dd, J = 9.5, 2.0 Hz, 1H), 4.11 (s, 3H). [M+H]+= 380.068 |
| **213** | 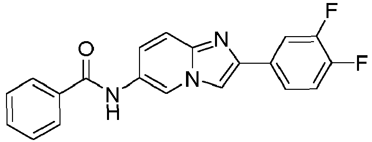 | N-(2-(3,4-difluorophenyl)imidazo[1,2-a]pyridin-6-yl)benzamide | 1H NMR (300 MHz, CDCl3) δ 9.35 (s, 1H), 7.95 - 7.83 (m, 3H), 7.82 - 7.73 (m, 1H), 7.71 (s, 1H), 7.67 - 7.48 (m, 5H), 7.24 - 7.16 (m, 1H), 6.99 (dd, J = 9.6, 2.1 Hz, 1H). [M+H]+= 349.71 |
| **214** | 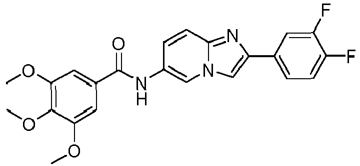 | N-(2-(3,4-difluorophenyl)imidazo[1,2-a]pyridin-6-yl)-3,4,5-trimethoxybenzamide | 1H NMR (300 MHz, CDCl3) δ 9.29 (s, 1H), 7.85 (s, 1H), 7.78 (dd, J = 11.3, 8.0 Hz, 1H), 7.69 - 7.56 (m, 3H), 7.19 (d, J = 8.5 Hz, 1H), 7.09 (s, 2H), 7.05 - 6.98 (m, 1H), 3.96 (s, 6H), 3.93 (s, 3H). [M+H]+= 440.05 |
| **219** | 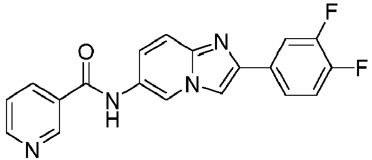 | N-(2-(3,4-difluorophenyl)imidazo[1,2-a]pyridin-6-yl)nicotinamide | [M+H]+= 351.06 |
| **220** | 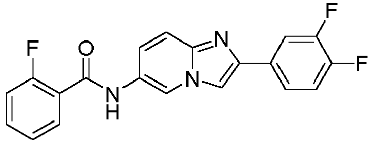 | N-(2-(3,4-difluorophenyl)imidazo[1,2-a]pyridin-6-yl)-2-fluorobenzamide | 1H NMR (300 MHz, DMSO) δ 10.62 (s, 1H), 9.34 (d, J = 1.9 Hz, 1H), 8.56 (s, 1H), 8.00 - 7.87 (m, 1H), 7.77 (s, 1H), 7.72 (t, J = 7.1 Hz, 1H), 7.62 (d, J = 9.4 Hz, 2H), 7.56 - 7.45 (m, 1H), 7.43 - 7.31 (m, 3H). [M+H]+= 368.05 |
| **221** | 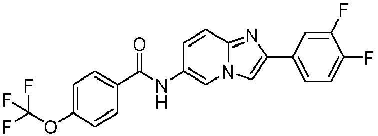 | N-(2-(3,4-difluorophenyl)imidazo[1,2-a]pyridin-6-yl)-4-(trifluoromethoxy)benzamide | 1H NMR (300 MHz, CDCl3) δ 9.31 (s, 1H), 7.95 (d, J = 8.7 Hz, 2H), 7.86 (s, 1H), 7.77 (t, J = 9.8 Hz, 1H), 7.73 - 7.56 (m, 3H), 7.37 (d, J = 8.3 Hz, 2H), 7.21 (t, J = 9.4 Hz, 1H), 6.99 (d, J = 9.4 Hz, 1H). [M+H]+= 434.02 |
| **222** | 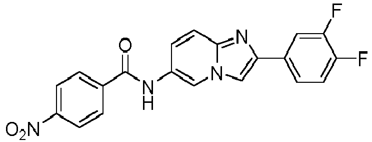 | N-(2-(3,4-difluorophenyl)imidazo[1,2-a]pyridin-6-yl)-4-nitrobenzamide | 1H NMR (300 MHz, DMSO) δ 10.74 (s, 1H), 9.35 (s, 1H), 8.58 (s, 1H), 8.41 (d, J = 8.5 Hz, 2H), 8.22 (d, J = 8.5 Hz, 2H), 8.00 - 7.87 (m, 1H), 7.79 (s, 1H), 7.65 (d, J = 9.5 Hz, 1H), 7.50 (t, J = 10.2 Hz, 2H). [M+H]+= 394.76 |
| | | | |
| **223** | 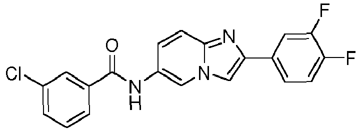 | 3-chloro-N-(2-(3,4-difluorophenyl)imidazo[1,2-a]pyridin-6-yl)benzamide | 1H NMR (300 MHz, DMSO) δ 10.51 (s, 1H), 9.32 (s, 1H), 8.57 (s, 1H), 8.04 (t, J = 1.9 Hz, 1H), 7.93 (t, J = 7.9 Hz, 2H), 7.78 (s, 1H), 7.73 - 7.68 (m, 1H), 7.66 - 7.56 (m, 2H), 7.55 - 7.42 (m, 2H). [M+H]+= 384.14 |
| **224** | 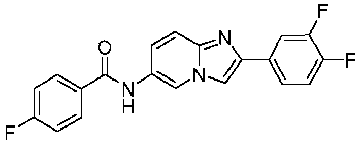 | N-(2-(3,4-difluorophenyl)imidazo[1,2-a]pyridin-6-yl)-4-fluorobenzamide | 1H NMR (300 MHz, DMSO) δ 10.42 (s, 1H), 9.32 (d, J = 1.8 Hz, 1H), 8.55 (s, 1H), 8.07 (dd, J = 8.7, 5.6 Hz, 2H), 7.94 (dd, J = 11.6, 8.4 Hz, 1H), 7.78 (d, J = 6.4 Hz, 1H), 7.62 (d, J = 9.6 Hz, 1H), 7.54 - 7.37 (m, 4H). [M+H]+= 368.05 |
| **225** | 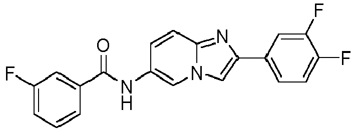 | N-(2-(3,4-difluorophenyl)imidazo[1,2-a]pyridin-6-yl)-3-fluorobenzamide | 1H NMR (300 MHz, DMSO) δ 10.48 (s, 1H), 9.33 (t, J = 1.4 Hz, 1H), 8.56 (s, 1H), 7.94 (ddd, J = 12.2, 7.9, 2.0 Hz, 1H), 7.88 - 7.75 (m, 3H), 7.68 - 7.58 (m, 2H), 7.50 (ddd, J = 9.7, 8.1, 2.6 Hz, 3H). [M+H]+= 368.05 |
| **226** | 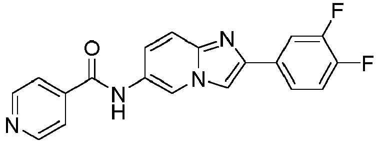 | N-(2-(3,4-difluorophenyl)imidazo[1,2-a]pyridin-6-yl)isonicotinamide | 1H NMR (300 MHz, DMSO) δ 10.68 (s, 1H), 9.33 (d, J = 1.9 Hz, 1H), 8.72 (s, 1H), 8.57 (s, 1H), 7.99 - 7.85 (m, 3H), 7.76 (d, J = 5.6 Hz, 2H), 7.64 (d, J = 9.7 Hz, 1H), 7.57 - 7.45 (m, 2H). [M+H]+= 351.06 |
| **227** | 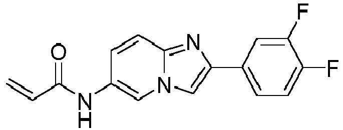 | N-(2-(3,4-difluorophenyl)imidazo[1,2-a]pyridin-6-yl)acrylamide | 1H NMR (300 MHz, DMSO) δ 10.35 (s, 1H), 9.31 (d, J = 1.9 Hz, 1H), 8.53 (s, 1H), 7.99 - 7.87 (m, 1H), 7.77 (s, 1H), 7.64 - 7.44 (m, 2H), 7.23 (dd, J = 9.6, 2.0 Hz, 1H), 6.45 (dd, J = 16.9, 9.9 Hz, 1H), 6.30 (dd, J = 17.0, 2.1 Hz, 1H), 5.81 (dd, J = 9.9, 2.2 Hz, 1H). [M+H]+= 300.11 |
| **216** | 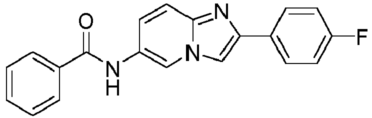 | N-(2-(4-fluorophenyl)imidazo[1,2-a]pyridin-6-yl)benzamide CAS number: 900534-25-0 | 1H NMR (300 MHz, CDCl3) δ 9.34 (s, 1H), 7.96 - 7.87 (m, 4H), 7.86 (s, 1H), 7.72 (s, 1H), 7.64 - 7.50 (m, 4H), 7.13 (t, J = 8.7 Hz, 2H), 6.98 (d, J = 9.6 Hz, 1H). [M+H]+= 332.09 |
| **217** | 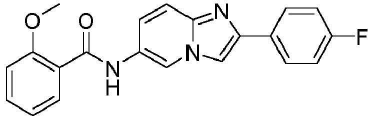 | N-(2-(4-fluorophenyl)imidazo[1,2-a]pyridin-6-yl)-2-methoxybenzamide CAS number: 2117850-26-5 | 1H NMR (300 MHz, CDCl3) δ 9.85 (s, 1H), 9.45 (s, 1H), 8.30 (dd, J = 7.9, 1.9 Hz, 1H), 7.92 (dd, J = 8.7, 5.5 Hz, 2H), 7.84 (s, 1H), 7.62 - 7.51 (m, 2H), 7.21 - 7.07 (m, 4H), 6.94 (dd, J = 9.5, 2.0 Hz, 1H), 4.11 (s, 3H). [M+H]+= 362.07 |
| **229** | 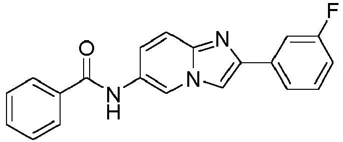 | N-(2-(3-fluorophenyl)imidazo[1,2-a]pyridin-6-yl)benzamide | 1H NMR (300 MHz, DMSO) δ 10.42 (s, 1H), 9.35 (d, J = 1.8 Hz, 1H), 8.59 (s, 1H), 8.05 - 7.93 (m, 2H), 7.76 (dd, J = 18.0, 8.9 Hz, 2H), 7.67 - 7.43 (m, 6H), 7.14 (t, J = 8.5 Hz, 1H). [M+H]+= 332.10 |
| **230** | 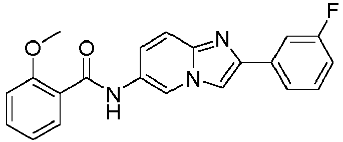 | N-(2-(3-fluorophenyl)imidazo[1,2-a]pyridin-6-yl)-2-methoxybenzamide | 1H NMR (300 MHz, DMSO) δ 10.38 (s, 1H), 9.33 (d, J = 1.9 Hz, 1H), 8.59 (s, 1H), 7.77 (dd, J = 16.6, 9.6 Hz, 2H), 7.66 - 7.44 (m, 6H), 7.17 (ddd, J = 14.7, 8.3, 4.6 Hz, 2H), 3.85 (s, 3H). [M+H]+= 362.08 |
| **133** | 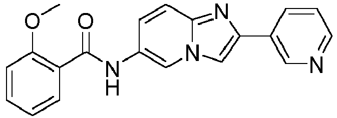 | 2-methoxy-N-(2-(pyridin-3-yl)imidazo[1,2-a]pyridin-6-yl)benzamide | 1H NMR (300 MHz, DMSO-d6) δ 10.29 (s, 1H), 9.39 (s, 1H), 9.15 (s, 1H), 8.63 (s, 1H), 8.51 (s,1H), 8.28 (d, J = 8.0 Hz, 1H), 7.64 (t, J = 7.3 Hz, 2H), 7.49 (dd, J = 14.6, 9.6 Hz, 2H), 7.36 (d, J = 9.4 Hz, 1H), 7.21 (d, J = 8.6 Hz, 1H), 7.09 (t, J = 7.3 Hz, 1H), 3.91 (s, 3H). [M+H]+ = 345.4 |
| **161** | 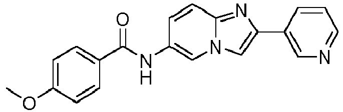 | 4-methoxy-N-(2-(pyridin-3-yl)imidazo[1,2-a]pyridin-6-yl)benzamide | [M+H]+ = 345.11 |
| **77** | 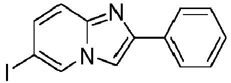 | 6-iodo-2-phenylimidazo[1,2-a]pyridine CAS number: 61982-63-6 | 1H NMR (300 MHz, DMSO-d6) δ 8.91 (s, 1H), 8.32 (s, 1H), 7.95 (d, J = 7.2 Hz, 2H), 7.51 - 7.39 (m, 4H), 7.33 (t, J = 7.2 Hz, 1H). [M+H]+ = 321.0 |
| **91** | 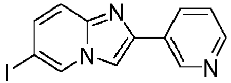 | 6-iodo-2-(pyridin-3-yl)imidazo[1,2-a]pyridine CAS number: 2001284-10-0 | 1H NMR (300 MHz, DMSO-d6) δ 9.15 (d, J = 2.2 Hz, 1H), 8.93 (d, J = 1.5 Hz, 1H), 8.53 (dd, J = 4.8, 1.6 Hz, 1H), 8.43 (s, 1H), 8.29 (dd, J = 8.0, 2.0 Hz, 1H), 7.54 - 7.38 (m, 3H). [M+H]+ = 321.9 |
| **211** | 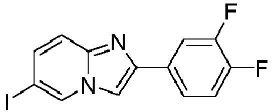 | 2-(3,4-difluorophenyl)-6-iodoimidazo[1,2-a]pyridine CAS number: 1450733-79-5 | 1H NMR (300 MHz, CDCl3) δ 8.42-8.36 (m, 1H), 7.81-7.71 (m, 2H), 7.64 (ddt, J = 8.1, 3.8, 1.7 Hz, 1H), 7.45-7.33 (m, 2H), 7.25-7.16 (m, 1H). [M+H]+= 356.89 |
| **215** | 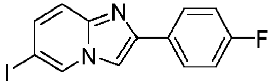 | 2-(4-fluorophenyl)-6-iodoimidazo[1,2-a]pyridine CAS number: 478040-44-7 | 1H NMR (300 MHz, CDCl3) δ 8.38 (s, 1H), 7.96 - 7.84 (m, 2H), 7.75 (s, 1H), 7.45 - 7.30 (m, 2H), 7.13 (t, J = 8.7 Hz, 2H). [M+H]+= 338.45 |
| **228** | 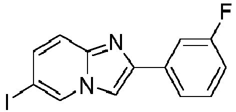 | 2-(3-fluorophenyl)-6-iodoimidazo[1,2-a]pyridine CAS number: 878979-11-4 | 1H NMR (300 MHz, DMSO) δ 9.13 (s, 1H), 8.58 (s, 1H), 7.91 - 7.74 (m, 3H), 7.67 - 7.53 (m, 2H), 7.31 (td, J = 8.3, 2.5 Hz, 1H) [M+H]+= 338.45 |
| **218** | 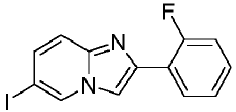 | 2-(2-fluorophenyl)-6-iodoimidazo[1,2-a]pyridine | 1H NMR (300 MHz, DMSO) δ 9.18 (s, 1H), 8.48 (d, J = 3.5 Hz, 1H), 8.17 - 8.05 (m, 1H), 7.88 (d, J = 9.4 Hz, 1H), 7.67 (d, J = 9.4 Hz, 1H), 7.59 - 7.38 (m, 3H). [M+H]+= 338.45 |

### Synthesis

The following general scheme may be implemented for obtaining a variety of compounds of formula (I'), (I), (II), (III) or (IV), starting from a compound of formula (1), as set out in scheme 1 herein after:

The synthesis is based on the condensation reaction of a substituted 2-aminopyridine of formula (**1**) with the carbonyl group of a substituted 2-bromoacetophenone of formula (**2**) followed by intramolecular cyclization to generate the imidazopyridine intermediate of formula (**I'**).according to route (A), following the incorporation of benzamide group through the Cu-catalytic coupling between halogenated compound of formula (**I'**) and a benzamide derivative of formula (**3**) according to route (B), or following the incorporation of amine group through the Pd-catalytic coupling between halogenated compound of formula (**I'**) and an amine derivative according to route (C), or following the incorporation of urea group through the Pd-catalytic coupling between halogenated compound of formula (**I'**) and an urea derivative according to route (D). The reaction between a carboxylic acid derivative of formula (I') and an amine derivative via a peptidic coupling follows to incorporate a reversed amide group (in comparison to the molecule of formula (**I**), (**II**), (**III**) and (**IV**)) according to route (E).

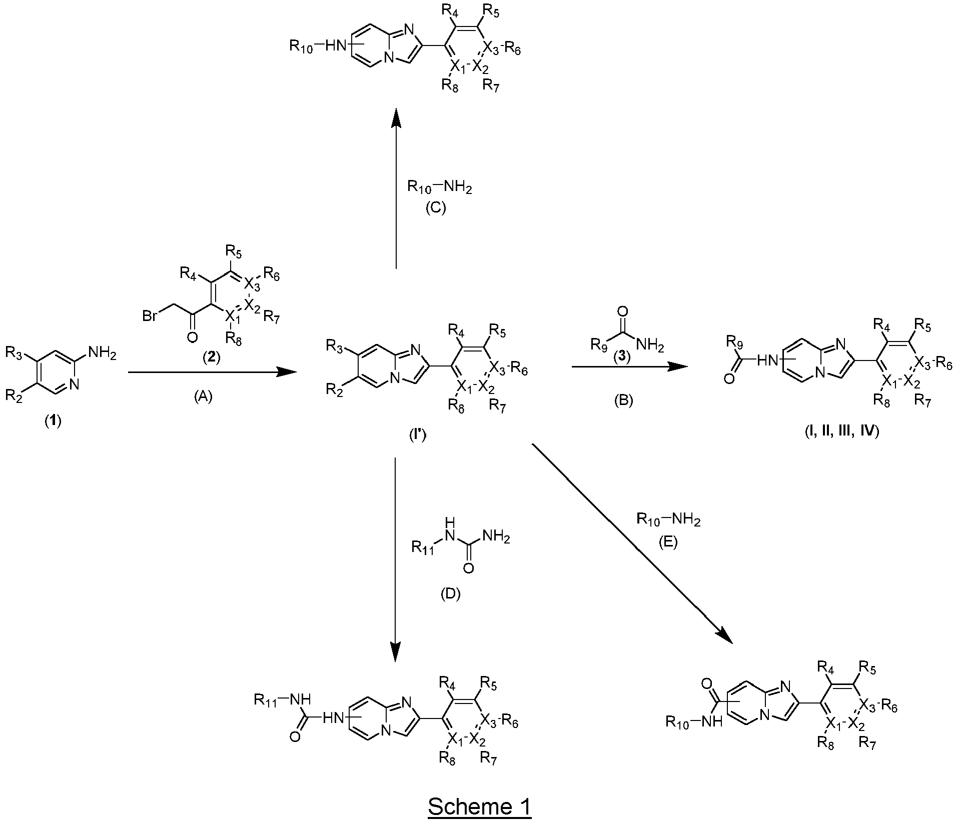

### Route (A)

According to route (A), the compound of formula (**1**), wherein R₂ or R₃ is a halogen atom, such as an iodine may be placed in an aprotic polar solvent, such as acetone. A 2-bromoacetophenone compound (**2**), wherein X₁, X₂ and X₃ are a carbon atom, may then be added for example in a molar ratio ranging from 0.8 to 1.2 with respect to the compound of formula (**1**). The reaction mixture can be heated at a temperature ranging from 40°C to 70°C, for example at 60°C and stirred for a time for example ranging from 4 to 18 hours, for example during 12 hours. Then the formed precipitate can be filtrated, washed by an aprotic polar solvent, such as acetone and dried under vacuum to give product (**I'**), where X₁, X₂, X₃ are a carbon atom and R₂ or R₃ is a halogen atom.

According to route (A), the compound of formula (**1**), wherein R₂ or R₃ is a halogen atom, such as an iodine may be placed in a protic polar solvent, such as ethanol. A 2-bromoacetophenone compound (**2**), wherein X₁, X₂ or X₃ is a nitrogen atom, may then be added for example in a molar ratio ranging from 0.8 to 1 with respect to the compound of formula (**1**) in presence of an inorganic base such as NaHCO₃ for example in a molar ratio ranging from 1 to 2 with respect to the compound of formula (**1**). The reaction mixture can be heated at a temperature ranging from 60°C to 100°C, for example at 80°C and stirred for a time for example ranging from 4 to 18 hours, for example during 12 hours. The reaction mixture can be cooled and poured on ice to precipitate the compound of formula (**I'**). Then the precipitate can be washed by water and diethyl ether and dried under vacuum overnight. Or, the reaction mixture can be concentrated under reduce pressure and the resulting residue can purified by column chromatography on silica gel to give product (**I'**), where X₁, X₂ or X₃ is a nitrogen atom and R₂ or R₃ is a halogen atom.

### Route (B)

According to route (B), the compound of formula (**I'**), wherein R₂ or R₃ is a halogen atom, such as an iodine may be placed in a non-polar solvent, such as toluene. The benzamide compound of formula (**3**), may then be added for example in a molar ratio ranging from 1 to 1.5 with respect to the compound of formula (**I**) in presence of an inorganic base, such as K₃PO₄ for example in a molar ratio ranging from 1.5 to 3 with respect to the compound of formula (**I**), in the presence of copper catalyst such as copper(I) iodide in amount ranging from 10 mol% to 25 mol% relative to the total amount of compound of formula (**I'**), and in the presence of ligand, such as trans-N,N'-dimethylcyclohexane-1,2-diamine in an amount ranging from 10 mol% to 25 mol% relative to the total amount of compound of formula (**I'**). The reaction mixture can then be heated at a temperature ranging from 100°C to 160°C, for example at 120°C and stirred for a time for example ranging from 15 to 72 hours, for example during 48 hours in sealed tube under inert gas and for example argon. The reaction mixture can be diluted with an organic solvent such as dichloromethane and filtrated through a celite pad. The filtrate can be concentrated under reduced pressure. The resulting residue can be purified by column chromatography on silica gel to give product (**I**), (**II**), (**III**) or (**IV**). The starting compound of formula (**3**) are available or can be prepared according to methods known to the person skilled in the art.

The expressions "between ... and ..." and "ranging from ... to ..." should be understood as meaning limits included, unless otherwise specified.

### Method for inducing immune cells

In one embodiment, the immune cells of the invention are selected among T lymphocytes, B lymphocytes, dendritic cells, natural killer cells, innate lymphoid cells, mesenchymal cells and myeloid cells. In a particular embodiment, the immune cells are T lymphocytes. In a particular embodiment, the immune cells are B lymphocytes. In a particular embodiment, the immune cells are myeloid cells.

Another aspect of the invention relates to a method for inducing the production of proteins, in particular interleukin-10, by immune cells characterized in that said immune cells are contacting with at least one compound inducing production of interleukin-10 of formula (I) as described above.

In one embodiment, immune cells were cultivated for two consecutive days in the presence of anti-IgM plus CpG in the presence of at least one compound of formula (I) at 0,25µM final concentration inducing the production of interleukin-10.

As used herein, the expression "immune cells induced by at least a compound of formula (I) or "induced immune cells" or "immune cells capable of producing interleukin 10" refers to immune cells having being in contact with at least a compound of formula (I) and after their incubation with such compound of formula (I) enabled to produce interleukin-10 (IL-10).

In one embodiment, the present invention also provides immune cells capable of producing interleukin 10 obtained by the method as defined above.

Another object of the invention relates to the immune cells induced by at least a compound of formula (I) as defined above for use thereof as a medicament. In particular, the present invention relates to the immune cells induced by at least a compound of formula (I) as defined above for use thereof in the prevention and/or the treatment of neurodegenerative diseases, inflammatory diseases, allergic diseases, adverse events related to transplantation, autoimmune diseases, metabolic diseases with an inflammatory component, inflammation facilitating cancer development or progression, and aging.

According to a particularly advantageous embodiment of the invention, the immune cells induced by at least a compound of formula (I) as defined above is particularly useful for preventing and/or the treating neurodegenerative diseases. The term "neurodegenerative disease", as used herein, refers to a condition or disorder in which neuronal cells are lost due to cell death bringing about a deterioration of cognitive functions or result in damage, dysfunction, or complications that may be characterized by neurological, neurodegenerative, physiological, psychological, or behavioral aberrations. Neurodegenerative diseases include, without limitation, multiple sclerosis, autistic spectrum disorder, depression, age-related macular degeneration, Creutzfeldt-Jakob disease, Alzheimer's Disease, radiotherapy induced dementia, axon injury, acute cortical spreading depression, alpha-synucleinopathies, brain ischemia, Huntington's disease, Guillain-Barre syndrome, permanent focal cerebral ischemia, peripheral nerve regeneration, post-status epilepticus model, spinal cord injury, sporadic amyotrophic lateral sclerosis, transmissible spongiform encephalopathy, myasthenia gravis, obsessive-compulsive disorder, optic neuritis, retinal degeneration, dry eye syndrome DES, Sjogren's syndrome, chronic idiopathic demyelinating disease (CID), anxiety, compulsive disorders (such as compulsive obsessive disorders), meningitis, neuroses, psychoses, insomnia and sleeping disorder, sleep apnoea, and drug abuse.

According to a particularly advantageous embodiment of the invention, the immune cells induced by at least a compound of formula (I) as defined above is particularly useful for preventing and/or the treating inflammatory diseases. Inflammatory diseases can include inflammatory diseases of the digestive apparatus, especially including the intestine (and particularly the colon in the case of irritable bowel syndrome, ulcero-haemorrhagic rectocolitis or Crohn's disease); pancreatitis, hepatitis (acute and chronic), inflammatory bladder pathologies and gastritis; inflammatory diseases of the locomotor apparatus, including rheumatoid arthritis, osteoarthritis, osteoporosis, traumatic arthritis, post-infection arthritis, muscular degeneration and dermatomyositis; inflammatory diseases of the urogenital apparatus and especially glomerulonephritis; inflammatory diseases of the cardiac apparatus and especially pericarditis and myocarditis and diseases including those for which inflammation is an underlying factor (such as atherosclerosis, transplant atherosclerosis, peripheral vascular diseases, inflammatory vascular diseases, intermittent claudication or limping, restenosis, strokes, transient ischaemic attacks, myocardial ischaemia and myocardial infarction), hypertension, hyperlipidaemia, coronary diseases, unstable angina (or angina pectoris), thrombosis, platelet aggregation induced by thrombin and/or the consequences of thrombosis and/or of the formation of atheroma plaques; inflammatory diseases of the respiratory and ORL apparatus, especially including asthma, acute respiratory distress syndrome, hayfever, allergic rhinitis and chronic obstructive pulmonary disease; inflammatory diseases of the skin, and especially urticaria, scleroderma, contact dermatitis, atopic dermatitis, psoriasis, ichthyosis, acne and other forms of folliculitis, rosacea and alopecia; the treatment of pain, irrespective of its origin, such as post-operative pain, neuromuscular pain, headaches, cancer-related pain, dental pain or osteoarticular pain; modulating pigmentation, for the treatment of: diseases with pigmentation disorders and especially benign dermatoses such as vitiligo, albinism, melasma, lentigo, ephelides, melanocytic naevus and all post-inflammatory pigmentations; and also pigmented tumours such as melanomas and their local (permeation nodules), regional or systemic metastases; photo-protection for the purpose of preventing:the harmful effects of sunlight, such as actinic erythema, cutaneous ageing, skin cancer (spinocellular, basocellular and melanoma) and especially diseases that accelerate its occurrence (xeroderma pigmentosum, basocellular naevus syndrome and familial melanoma); photodermatoses caused by exogenous photosensitizers and especially those caused by contact photosensitizers (for example furocoumarins, halogenated salicylanilides and derivatives, and local sulfamides and derivatives) or those caused by systemic photosensitizers (for example psoralenes, tetracyclines, sulfamides, phenothiazines, nalidixic acid and tricyclic antidepressants); bouts or outbreaks of dermatosis with photosensitivity and especiallylight-aggravated dermatoses (for example lupus erythematosus, recurrent herpes, congenital poikilodermal or telangiectatic conditions with photosensitivity (Bloom's syndrome, Cockayne's syndrome or Rothmund-Thomson syndrome), actinic lichen planus, actinic granuloma, superficial disseminated actinic porokeratosis, acne rosacea, juvenile acne, bullous dermatosis, Darier's disease, lymphoma cutis, psoriasis, atopic dermatitis, contact eczema, Chronic Actinic Dermatosis (CAD), follicular mucinosis, erythema multiforme, fixed drug eruption, cutaneous lymphocytoma, reticular erythematous mucinosis, and melasma);dermatoses with photosensitivity by deficiency of the protective system with anomalies of melanin formation or distribution (for example oculocutaneous albinism, phenylketonuria, hypopituitarism, vitiligo and piebaldism) and with deficiency of the DNA repair systems (for example xeroderma pigmentosum and Cockayne's syndrome), dermatoses with photosensitivity via metabolic anomalies, for instance cutaneous porphyria (for example tardive cutaneous porphyria, mixed porphyria, erythropoietic protoporphyria, congenital erythropoietic porphyria (Günther's disease), and erythropoietic coproporphyria), pellagra or pellagroid erythema (for example pellagra, pellagroid erythemas and tryptophan metabolism disorders); bouts or outbreaks of idiopathic photodermatoses and especially PMLE (polymorphic light eruption), benign summer light eruption, actinic prurigo, persistent photosensitizations (actinic reticuloid, remanent photosensitizations and photosensitive eczema), solar urticaria, hydroa vacciniforme, juvenile spring eruption and solar pruritus;modifying the colour of the skin or head hair and bodily hair, and especially by tanning the skin by increasing melanin synthesis or bleaching it by interfering with melanin synthesis, but also by preventing the bleaching or greying of head hair or bodily hair (for example canities and piebaldism); and also modifying the colour of head hair and bodily hair in cosmetic indications; modifying the sebaceous functions, and especially the treatment of: hyperseborrhoea complaints and especially acne, seborrhoeic dermatitis, greasy skin and greasy hair, hyperseborrhoea in Parkinson's disease and epilepsy and hyperandrogenism; complaints with reduction of sebaceous secretion and especially xerosis and all forms of dry skin; benign or malignant proliferation of sebocytes and the sebaceous glands; inflammatory complaints of the pilosebaceous follicles and especially acne, boils, anthrax and folliculitis; male or female sexual dysfunctions; male sexual dysfunctions including, but not limited to, impotence, loss of libido and erectile dysfunction; female sexual dysfunctions including, but not limited to, sexual stimulation disorders or desire-related disorders, sexual receptivity, orgasm, and disturbances of the major points of sexual function; pain, premature labour, dysmenorrhoea, excessive menstruation, and endometriosis; disorders related to weight but not limited to obesity and anorexia (such as modification or impairment of appetite, metabolism of the spleen, or the vocable irreproachable taking of fat or carbohydrates); diabetes mellitus (by tolerance to glucose doses and/or reduction of insulin resistance); cancer and in particular lung cancer, prostate cancer, bowel cancer, breast cancer, ovarian cancer, bone cancer or angiogenesis disorders including the formation or growth of solid tumours.

According to a particularly advantageous embodiment of the invention, the immune cells induced by at least a compound of formula (I) as defined above is particularly useful for preventing and/or the treating immune-mediated diseases. Immune-mediated diseases include, for example, but not limited to, asthma, allergies, arthritis (e.g., rheumatoid arthritis, psoriatic arthritis, and ankylosing spondylitis), juvenile arthritis, inflammatory bowel diseases (e.g., ulcerative colitis and Crohn's disease), endocrinopathies (e.g., type 1 diabetes and Graves' disease), vascular diseases (e.g., autoimmune hearing loss, systemic vasculitis, and atherosclerosis), and skin diseases (e.g., acne vulgaris dermatomyositis, pemphigus, systemic lupus erythematosus (SLE), discoid lupus erthematosus, scleroderma, psoriasis, plaque psoriasis, vasculitics, vitiligo and alopecias). Hashimoto's thyroiditis, pernicious anemia, Cushing's disease, Addison's disease, chronic active hepatitis, polycystic ovary syndrome (PCOS), celiac disease, pemphigus, transplant rejection (allograft transplant rejection), graft-versus-host disease (GVDH).

According to a particularly advantageous embodiment of the invention, the immune cells induced by at least a compound of formula (I) as defined above is particularly useful for preventing and/or the treating allergic diseases. As used herein, the term "allergy" or "allergies" or "allergic reaction" or "allergic response" refers to a disorder (or improper reaction) of the immune system. Allergic reactions occur to normally harmless environmental substances known as allergens; these reactions are acquired, predictable, and rapid. Strictly, allergy is one of four forms of hypersensitivity and is called type I (or immediate) hypersensitivity. It is characterized by excessive activation of certain white blood cells called mast cells and basophils by a type of antibody known as IgE, resulting in an extreme inflammatory response. Common allergic reactions include eczema, hives, hay fever, asthma, food allergies, and reactions to the venom of stinging insects such as wasps and bees. Mild allergies like hay fever are highly prevalent in the human population and cause symptoms such as allergic conjunctivitis, itchiness, and runny nose. Allergies can play a major role in conditions such as asthma. In some people, severe allergies to environmental or dietary allergens or to medication may result in life-threatening anaphylactic reactions and potentially death. The treatment of allergies via immune suppression is therefore one embodiment of the present invention.

According to a particularly advantageous embodiment of the invention, the immune cells induced by at least a compound of formula (I) as defined above is particularly useful for preventing and/or the treating adverse events related to transplantation. Adverse events related to transplantation relate to any medical condition surrounding rejection of transplanted material, or an immune response caused by transplantation of tissue to a recipient subject. Advantageously, adverse events related to transplantation can be directed to the kidney, liver, heart, lung, pancreas, bone marrow, cornea, intestine or skin (skin allograft, homograft or heterograft, etc.).

According to a particularly advantageous embodiment of the invention, the immune cells induced by at least a compound of formula (I) as defined above is particularly useful for preventing and/or the treating autoimmune diseases. Examples of autoimmune disease include, but are not limited to arthritis (rheumatoid arthritis, juvenile-onset rheumatoid arthritis, osteoarthritis, psoriatic arthritis, and ankylosing spondylitis), psoriasis, dermatitis including atopic dermatitis, chronic idiopathic urticaria, including chronic autoimmune urticaria, polymyositis/ dermatomyositis, toxic epidermal necrolysis, scleroderma (including systemic scleroderma), sclerosis such as progressive systemic sclerosis, inflammatory bowel disease (IBD) (for example, Crohn's disease, ulcerative colitis, autoimmune inflammatory bowel disease), pyoderma gangrenosum, erythema nodosum, primary sclerosing cholangitis, episcleritis), respiratory distress syndrome, including adult respiratory distress syndrome (ARDS), meningitis, IgE-mediated diseases such as anaphylaxis and allergic and atopic rhinitis, encephalitis such as Rasmussen's encephalitis, uveitis or autoimmune uveitis, colitis such as microscopic colitis and collagenous colitis, glomerulonephritis (GN) such as membranous GN (membranous nephropathy), idiopathic membranous GN, membranous proliferative GN (MPGN), including Type I and Type II, and rapidly progressive GN, allergic conditions, allergic reaction, eczema, asthma, conditions involving infiltration of T cells and chronic inflammatory responses, atherosclerosis, autoimmune myocarditis, leukocyte adhesion deficiency, systemic lupus erythematosus (SLE) such as cutaneous SLE, subacute cutaneous lupus erythematosus, lupus (including nephritis, cerebritis, pediatric, non-renal, discoid, alopecia), juvenile onset (Type I) diabetes mellitus, including pediatric insulin-dependent diabetes mellitus (IDDM), adult onset diabetes mellitus (Type II diabetes), multiple sclerosis (MS) such as spino-optical MS, immune responses associated with acute and delayed hypersensitivity mediated by cytokines and T-lymphocytes, tuberculosis, sarcoidosis, granulomatosis including lymphomatoid granulomatosis, Wegener's granulomatosis, agranulocytosis, vasculitis (including large vessel vasculitis (including polymyalgia rheumatica and giant cell (Takayasu's) arteritis), medium vessel vasculitis (including Kawasaki's disease and polyarteritis nodosa), CNS vasculitis, systemic necrotizing vasculitis, and ANCA-associated vasculitis, such as Churg-Strauss vasculitis or syndrome (CSS)), temporal arteritis, aplastic anemia, Coombs positive anemia, Diamond Blackfan anemia, hemolytic anemia or immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, CNS inflammatory disorders, multiple organ injury syndrome, antigen-antibody complex mediated diseases, anti-glomerular basement membrane disease, antiphospholipid antibody syndrome, allergic neuritis, Bechet's or Behcet's disease, Castleman's syndrome, Goodpasture's syndrome, Reynaud's syndrome, Sjogren's syndrome, Stevens-Johnson syndrome, pemphigoid such as pemphigoid bullous, pemphigus (including vulgaris, foliaceus, and pemphigus mucus-membrane pemphigoid), autoimmune polyendocrinopathies, Reiter's disease, immune complex nephritis, chronic neuropathy such as IgM polyneuropathies or IgMmediated neuropathy, thrombocytopenia (as developed by myocardial infarction patients, for example), including thrombotic thrombocytopenic purpura (TTP) and autoimmune or immune-mediated thrombocytopenia such as idiopathic thrombocytopenic purpura (ITP) including chronic or acute ITP, autoimmune disease of the testis and ovary including autoimune orchitis and oophoritis, primary hypothyroidism, hypoparathyroidism, autoimmune endocrine diseases including thyroiditis such as autoimmune thyroiditis, chronic thyroiditis (Hashimoto's thyroiditis), or subacute thyroiditis, autoimmune thyroid disease, idiopathic hypothyroidism, Addison's disease, Grave's disease, polyglandular syndromes such as autoimmune polyglandular syndromes (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis, myasthenia gravis, cerebellar degeneration, limbic and/or brainstem encephalitis, neuromyotonia, opsoclonus or opsoclonus myoclonus syndrome (OMS), and sensory neuropathy, Sheehan's syndrome, autoimmune hepatitis, chronic hepatitis, lupoid hepatitis, chronic active hepatitis or autoimmune chronic active hepatitis, lymphoid interstitial pneumonitis, bronchiolitis obliterans (non-transplant) vs NSIP, GuillainBarre syndrome, Berger's disease (IgA nephropathy), primary biliary cirrhosis, celiac sprue (gluten enteropathy), refractory sprue, dermatitis herpetiformis, cryoglobulinemia, amyotrophic lateral sclerosis (ALS; Lou Gehrig's disease), coronary artery disease, autoimmune inner ear disease (AIED) or autoimmune hearing loss, opsoclonus myoclonus syndrome (OMS), polychondritis such as refractory polychondritis, pulmonary alveolar proteinosis, amyloidosis, giant cell hepatitis, scleritis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal segmental glomerulosclerosis (FSGS), endocrine ophthalmopathy, uveoretinitis, autoimmune hepatological disorder, fibromyalgia, multiple endocrine failure, Schmidt's syndrome, adrenalitis, gastric atrophy, presenile dementia, demyelinating diseases, Dressler's syndrome, alopecia areata, CREST syndrome (calcinosis, Raynaud's phenomenon, esophageal dysmotility, sclerodactyly, and telangiectasia), male and female autoimmune infertility, ankylosing spondolytis, mixed connective tissue disease, Chagas' disease, rheumatic fever, recurrent abortion, farmer's lung, erythema multiforme, post-cardiotomy syndrome, Cushing's syndrome, bird-fancier's lung, Alport's syndrome, alveolitis such as allergic alveolitis and fibrosing alveolitis, interstitial lung disease, transfusion reaction, leprosy, malaria, leishmaniasis, kypanosomiasis, schistosomiasis, ascariasis, aspergillosis, Sampter's syndrome, Caplan's syndrome, dengue, endocarditis, endomyocardial fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, or Fuch's cyclitis, Henoch-Schonlein purpura, human immunodeficiency virus (HIV) infection, echovirus infection, cardiomyopathy, Alzheimer's disease, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, and giant cell polymyalgia. The treatment of autoimmune disorders via immune suppression is therefore one embodiment of the present invention.

According to a particularly advantageous embodiment of the invention, the immune cells induced by at least a compound of formula (I) as defined above is particularly useful for preventing and/or the treating metabolic diseases with an inflammatory component, such as arteriosclerosis with resulting coronary heart diseases, adiposity or diabetes.

According to a particularly advantageous embodiment of the invention, the immune cells induced by at least a compound of formula (I) as defined above is particularly useful for preventing and/or the treating aging.

Advantageously, when administered to the patient, the immune cells induced by at least a compound of formula (I) rapidly enter the inflamed central nervous system (CNS) upon administration, where they suppressed the deleterious activation of microglia and CNS-associated macrophages. Thus, the immune cells induced by at least a compound of formula (I) suppress the deleterious activation of myeloid cells in tissues targeted by deleterious immune responses and inflammation.

### Pharmaceutical composition

Another object of the invention concerns a pharmaceutical composition comprising at least a dose of induced immune cell as defined above.

In some embodiments, the dose of immune cells is provided as a composition or formulation, such as a pharmaceutical composition or formulation. Such compositions can be used in accord with the provided methods and/or with the provided articles of manufacture or compositions, such as in the treatment of diseases, conditions, and disorders.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In an embodiment, the subject can be a human being, man or woman, regardless of age. In another embodiment, the patient can be a non-human animal, advantageously a non-human mammal, for example a dog, a cat, a mouse, a rat, a hamster, a rabbit, a chinchilla, a horse, a cow, a pig, a sheep, a goat or a primate.

In one embodiment, the pharmaceutical composition comprising a dose of immune cell induced by at least a compound of formula (I) as defined above, and at least one pharmaceutically acceptable carrier.

In a particularly advantageous embodiment, the pharmaceutical composition comprise a dose of immune cell induced by the compound 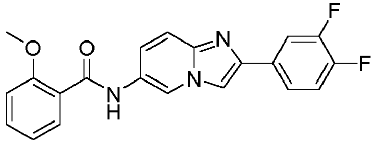 and at least one pharmaceutically acceptable carrier.

In a particularly advantageous embodiment, the pharmaceutical composition comprise a dose of immune cell induced by the compound 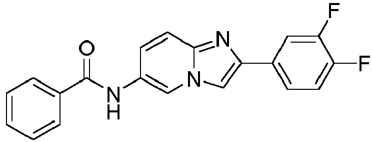 and at least one pharmaceutically acceptable carrier.

In a particularly advantageous embodiment, the pharmaceutical composition comprise a dose of immune cell induced by the compound 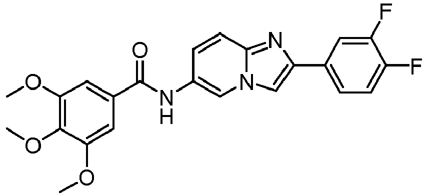 and at least one pharmaceutically acceptable carrier.

In a particularly advantageous embodiment, the pharmaceutical composition comprise a dose of immune cell induced by the compound 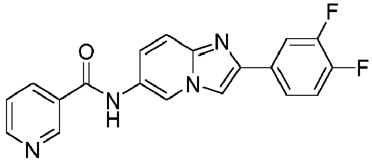 and at least one pharmaceutically acceptable carrier.

In a particularly advantageous embodiment, the pharmaceutical composition comprise a dose of immune cell induced by the compound 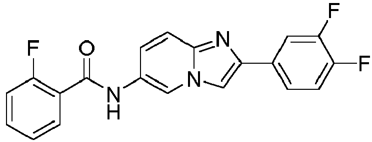 and at least one pharmaceutically acceptable carrier.

In a particularly advantageous embodiment, the pharmaceutical composition comprise a dose of immune cell induced by the compound 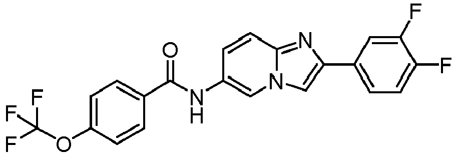 and at least one pharmaceutically acceptable carrier.

In a particularly advantageous embodiment, the pharmaceutical composition comprise a dose of immune cell induced by the compound 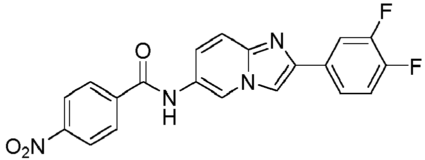 and at least one pharmaceutically acceptable carrier.

In a particularly advantageous embodiment, the pharmaceutical composition comprise a dose of immune cell induced by the compound 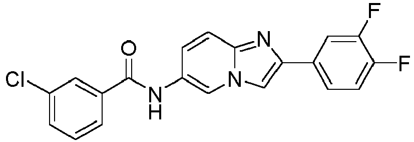 and at least one pharmaceutically acceptable carrier.

In a particularly advantageous embodiment, the pharmaceutical composition comprise a dose of immune cell induced by the compound 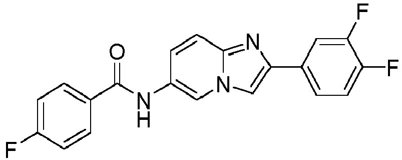 and at least one pharmaceutically acceptable carrier.

In a particularly advantageous embodiment, the pharmaceutical composition comprise a dose of immune cell induced by the compound 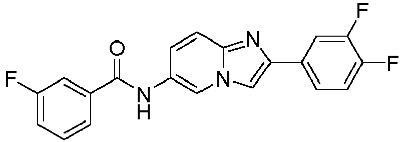 and at least one pharmaceutically acceptable carrier.

In a particularly advantageous embodiment, the pharmaceutical composition comprise a dose of immune cell induced by the compound 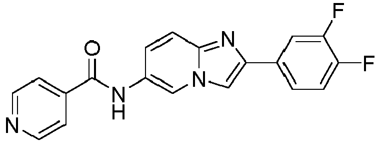 and at least one pharmaceutically acceptable carrier.

In a particularly advantageous embodiment, the pharmaceutical composition comprise a dose of immune cell induced by the compound 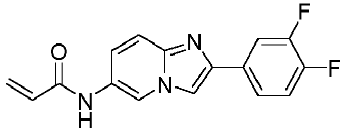 and at least one pharmaceutically acceptable carrier.

In a particularly advantageous embodiment, the pharmaceutical composition comprise a dose of immune cell induced by the compound 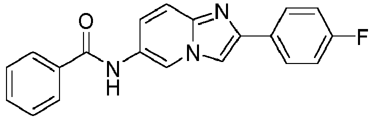 and at least one pharmaceutically acceptable carrier.

In a particularly advantageous embodiment, the pharmaceutical composition comprise a dose of immune cell induced by the compound 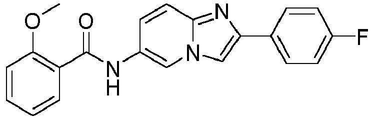 and at least one pharmaceutically acceptable carrier.

In a particularly advantageous embodiment, the pharmaceutical composition comprise a dose of immune cell induced by the compound 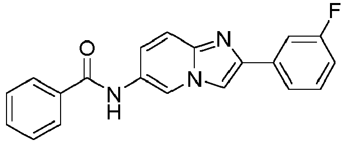 and at least one pharmaceutically acceptable carrier.

In a particularly advantageous embodiment, the pharmaceutical composition comprise a dose of immune cell induced by the compound 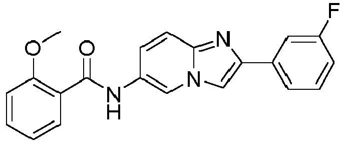 and at least one pharmaceutically acceptable carrier.

In a particularly advantageous embodiment, the pharmaceutical composition comprise a dose of immune cell induced by the compound 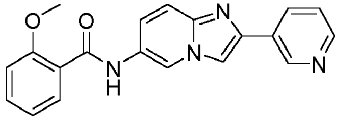 and at least one pharmaceutically acceptable carrier.

In a particularly advantageous embodiment, the pharmaceutical composition comprise a dose of immune cell induced by the compound 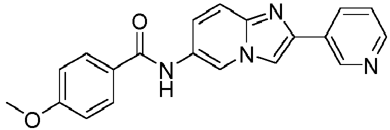 and at least one pharmaceutically acceptable carrier.

In a particularly advantageous embodiment, the pharmaceutical composition comprise a dose of immune cell induced by the compound 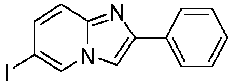 and at least one pharmaceutically acceptable carrier.

In a particularly advantageous embodiment, the pharmaceutical composition comprise a dose of immune cell induced by the compound 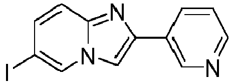 and at least one pharmaceutically acceptable carrier.

In a particularly advantageous embodiment, the pharmaceutical composition comprise a dose of immune cell induced by the compound 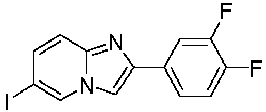 and at least one pharmaceutically acceptable carrier.

In a particularly advantageous embodiment, the pharmaceutical composition comprise a dose of immune cell induced by the compound 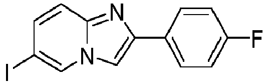 and at least one pharmaceutically acceptable carrier.

In a particularly advantageous embodiment, the pharmaceutical composition comprise a dose of immune cell induced by the compound 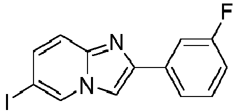 and at least one pharmaceutically acceptable carrier.

In a particularly advantageous embodiment, the pharmaceutical composition comprise a dose of immune cell induced by the compound 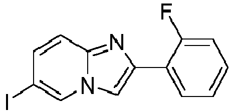 and at least one pharmaceutically acceptable carrier.

### Carrier

In some embodiments, the immune cells are formulated with a pharmaceutically acceptable carrier. In some aspects, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition.

The formulations can include aqueous solutions. The formulation or composition may also contain more than one active ingredient useful for the particular indication, disease, or condition being treated with the induced immune cells, where the respective activities do not adversely affect one another. Such second active ingredients are suitably present in combination in amounts that are effective for the purpose intended. In an advantageous embodiment, the pharmaceutical composition according to the invention also comprises at least a second active ingredient. In a particularly advantageous embodiment of the invention, the at least a second active ingredient can interact synergically with the immune cell induced by at least a compound of formula (I) according to the invention.

Examples of second active ingredient include, but are not limited to an estrogen receptor modulator; an androgen receptor modulator; a glucocorticoid, a retinoid receptor modulator; a cytotoxic agent; an anti-proliferative agent comprises: adriamycin, dexamethasone, vincristine, cyclophosphamide, fluorouracil, topotecan, taxol, interferons, or platinum derivatives; an anti-inflammatory agent comprises: corticosteroids, TNF blockers, IL-1 RA, azathioprine, cyclophosphamide, JAK inhibitors (e.g. tofacitinib and baricitinib), SYK inhibitors, BTK inhibitors or sulfasalazine; a prenyl-protein transferase inhibitor; an HMG-CoA reductase inhibitor; an HIV protease inhibitor; a reverse transcriptase inhibitor; an angiogenesis inhibitor comprises: sorafenib, sunitinib, pazopanib, or everolimus; an immunomodulatory or immunosuppressive agents comprises: cyclosporin, tacrolimus, rapamycin, mycophenolate mofetil, interferons, corticosteroids, cyclophophamide, azathioprine, or sulfasalazine; a PPAR-γ agonist comprising thiazolidinediones; a PPAR-δ agonist; an inhibitor of inherent multidrug resistance; an agent for the treatment of anemia, comprising: erythropoiesis-stimulating agents, vitamins, or iron supplements; an anti-emetic agent including 5-HT3 receptor antagonists, dopamine antagonists, NK1 receptor antagonist, H1 histamine receptor antagonists, cannabinoids, benzodiazepines, anticholinergic agents, or steroids; an agent for the treatment of neutropenia; an immunologic-enhancing agents; a proteasome inhibitors; an HDAC inhibitors; an inhibitor of the chemotrypsin-like activity in the proteasome; a E3 ligase inhibitors; a modulator of the immune system including interferon-alpha, Bacillus Calmette-Guerin (BCG), or ionizing radition (UVB) that can induce the release of cytokines, interleukins, TNF, or induce release of death receptor ligands including TRAIL; a modulator of death receptors TRAIL, or TRAIL agonists including humanized antibodies HGS-ETR1, or HGS-ETR2; neurotrophic factors selected from the group of: cetylcholinesterase inhibitors, MAO inhibitors, interferons, anti-convulsants, ion channel blockers, or riluzole; anti-Parkinsonian agents comprising anticholinergic agents, or dopaminergic agents, including dopaminergic precursors, monoamine oxidase B inhibitors, COMT inhibitors, dopamine receptor agonists; agents for treating cardiovascular disease comprises beta-blockers, ACE inhibitors, diuretics, nitrates, calcium channel blockers, or statins; agents for treating liver disease comprises corticosteroids, cholestyramine, or interferons; anti-viral agents, including nucleoside reverse transcriptase inhibitors, non-nucleoside reverse transcriptase inhibitors, protease inhibitors, integrase inhibitors, fusion inhibitors, chemokine receptor antagonists, polymerase inhibitors, viral proteins synthesis inhibitors, viral protein modification inhibitors, neuraminidase inhibitors, fusion or entry inhibitors; agents for treating blood disorders comprising: corticosteroids, anti-leukemic agents, or growth factors; agents for treating immunodeficiency disorders comprising gamma globulin, adalimumab, etarnecept, tocilizumab or infliximab; a HMG-CoA reductase inhibitors including torvastatin, fluvastatin, lovastatin, pravastatin, rosuvastatin, simvastatin, or pitavastatin, or in combination, or sequentially with radiation, or at least one chemotherapeutic agents e.g., asparaginase, busulfan, carboplatin, cisplatin, daunorubicin, doxorubicin, fluorouracil, gemcitabine, hydroxyurea, methotrexate, paclitaxel, rituximab, vinblastine and vincristine.

In an advantageous embodiment of the invention, when the pharmaceutical composition comprises a second active ingredient, the pharmaceutical composition is adapted for a simultaneous administration or a sequential administration of the immune cell induced by at least a compound of formula (I) as defined above and of the second active ingredient.

Within the meaning of the present invention, by "simultaneous administration" is meant an administration of the immune cell induced by at least a compound of formula (I) as defined above and of the second active ingredient at the same time, at the same moment, to a patient in therapeutically efficacious quantities in order to permit the synergic effect of the pharmaceutical composition. This does not necessarily mean that the immune cell induced by at least a compound of formula (I) as defined above and the second active ingredient must be administered in the form of a mixture; they can in fact be administered simultaneously but separately, in the form of distinct compositions.

By "present in two distinct compositions" is meant that the immune cell induced by at least a compound of formula (I) as defined above and the second active ingredient are physically separate. They are then implemented, administered separately in therapeutically efficacious quantities, to permit the synergic effect of the pharmaceutical composition, without prior mixing, in several (at least two) dosage forms (for example two distinct compositions).

In another particular embodiment of the invention, the immune cell induced by at least a compound of formula (I) as defined above and the second active ingredient can be present in one and the same composition in therapeutically efficacious quantities in order to permit the synergic effect of the pharmaceutical composition. Within the meaning of the present invention, by "present in one and the same composition" is meant the physical combination of the immune cell induced by at least a compound of formula (I) as defined above and the second active ingredient. The immune cell induced by at least a compound of formula (I) as defined above and the second active ingredient must then be administered simultaneously, as they are administered together in the form of a mixture, in one and the same dosage form and in therapeutically efficacious quantities, to permit the synergic effect of the pharmaceutical composition.

Within the meaning of the present invention, by the expression "sequential administration" is meant that the immune cell induced by at least a compound of formula (I) as defined above and the second active ingredient are administered in therapeutically efficacious quantities in order to permit the synergic effect of the pharmaceutical composition, not simultaneously but separately over time, one after another. The terms "precede" or "preceding" and "follow" or "following" then apply. The term "precede" or "preceding" is used when a component of the pharmaceutical composition according to the invention is administered a few minutes or a few hours, or even a few days before the administration of the other component(s) of the pharmaceutical composition. Conversely, the term "follow" or "following" is used when a component of the pharmaceutical composition according to the invention is administered a few minutes or a few hours, or even a few days after the administration of the other component(s) of the pharmaceutical composition. In a particularly advantageous embodiment of the invention, the immune cell induced by at least a compound of formula (I) as defined above are administered several days before the second active ingredient.

In an advantageous embodiment of the invention, when the pharmaceutical composition comprises a second active ingredient, the pharmaceutical composition is adapted for a sequential administration of the immune cell induced by at least a compound of formula (I) as defined above and of the second active ingredient.

The pharmaceutical composition in some embodiments comprises the induced immune cells in amounts effective to treat the disease or condition, such as a therapeutically effective or prophylactically effective amount. Within the meaning of the present invention, the terms "therapeutically efficacious" or "efficacious quantity for treatment", or "pharmaceutically efficacious" denote the quantity of immune cell induced by at least a compound of formula (I) as defined above necessary to inhibit or reverse a disease, advantageously for treating a disease selected among neurodegenerative disease, inflammatory disease, immune-mediated disease, allergic disease, adverse events related to transplantation, autoimmune disease, metabolic diseases with an inflammatory component, inflammation facilitating cancer development or progression, and aging. The determination of a therapeutically efficacious quantity depends specifically on factors such as the toxicity and efficacy of the medicament. These factors will differ as a function of other factors such as the activity, the relative bioavailability, the body weight of the patient, the severity of the adverse effects and the preferred administration route. The toxicity can be determined by using methods that are well known in the art. The same applies for the efficacy. A pharmaceutically efficacious quantity is consequently a quantity that is considered by the clinician as toxicologically tolerable but efficacious. Therapeutic efficacy in some embodiments is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition The induced immune cells of the invention may be administered using standard administration techniques, formulations, and/or devices. Provided are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition comprising the induced immune cells of the invention), it will generally be formulated in a unit dosage injectable form (solution, suspension, emulsion). Formulations include those for oral, intravenous, intraperitoneal, subcutaneous, pulmonary, transdermal, intradermal, intratumoral, intralesional, intramuscular, intranasal, buccal, sublingual, topical, local, otic, inhalational, retinal, intraocular, periocular, subretinal, intravitreal, or suppository administration, trans-septal, subscleral, intrachoroidal, intracameral, subconjectval, subconjuntival, sub-Tenon's, retrobulbar, peribulbar, or posterior juxtascleral administration. The term "parenteral," as used herein, includes intravenous, intramuscular, subcutaneous, rectal, vaginal, and intraperitoneal administration. In some embodiments, the agent or cell populations are administered to a subject using peripheral systemic delivery by intravenous, intraperitoneal, or subcutaneous injection.

Compositions in some embodiments are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the cells in a solvent, such as in admixture with a suitable carrier, diluent, or excipient such as sterile water, physiological saline, glucose, dextrose, or the like.

The formulations to be used for in vivo administration are generally sterile. Sterility may be readily accomplished, e.g., by filtration through sterile filtration membranes.

### Dosing

In some embodiments, a dose of induced immune cells of the invention is administered to subjects in accord with the provided methods, and/or with the provided articles of manufacture or compositions. In some embodiments, the size or timing of the doses is determined as a function of the particular disease or condition in the subject. In some cases, the size or timing of the doses for a particular disease in view of the provided description may be empirically determined.

In some embodiments, the dose of induced immune cells of the invention comprises between at or about 2×10⁵ of the cells/kg and at or about 9×10¹⁴ of the cells/kg, advantageously between at or about 2×10⁵ of the cells/kg and at or about 9×10¹³ of the cells/kg, advantageously between at or about 2×10⁵ of the cells/kg and at or about 9×10¹² of the cells/kg, advantageously, between at or about 2×10⁵ of the cells/kg and at or about 9×10¹¹ of the cells/kg, advantageously between at or about 2×10⁵ of the cells/kg and at or about 9×10¹⁰ of the cells/kg, advantageously between at or about 2×10⁵ of the cells/kg and at or about 9×10⁹ of the cells/kg, advantageously between at or about 2×10⁵ of the cells/kg and at or about 9×10⁸ of the cells/kg.

In some embodiments, the dose of induced immune cells of the invention comprises at least or at least about or at or about 2×10⁵ of the cells (e.g. IL10-expressing cells) per kilogram body weight of the subject (cells/kg), such as at least or at least about or at or about 3×10⁵ cells/kg, at least or at least about or at or about 4×10⁵ cells/kg, at least or at least about or at or about 5×10⁵ cells/kg, at least or at least about or at or about 6×10⁵ cells/kg, at least or at least about or at or about 7×10⁵ cells/kg, at least or at least about or at or about 8×10⁵ cells/kg, at least or at least about or at or about 9×10⁵ cells/kg, at least or at least about or at or about 1×10⁶ cells/kg, or at least or at least about or at or about 2×10⁶ cells/kg, at least or at least about or at or about 3×10⁶ cells/kg, at least or at least about or at or about 4×10⁶ cells/kg, at least or at least about or at or about 5×10⁶ cells/kg, at least or at least about or at or about 6×10⁶ cells/kg, at least or at least about or at or about 7×10⁶ cells/kg, at least or at least about or at or about 8×10⁶ cells/kg, at least or at least about or at or about 9×10⁶ cells/kg, at least or at least about or at or about 1×10⁷ cells/kg, or at least or at least about or at or about 2×10⁷ cells/kg, at least or at least about or at or about 3×10⁷ cells/kg, at least or at least about or at or about 4×10⁷ cells/kg, at least or at least about or at or about 5×10⁷ cells/kg, at least or at least about or at or about 6×10⁷ cells/kg, at least or at least about or at or about 7×10⁷ cells/kg, at least or at least about or at or about 8×10⁷ cells/kg, at least or at least about or at or about 9×10⁷ cells/kg, at least or at least about or at or about 1×10⁸ cells/kg, or at least or at least about or at or about 2×10⁸ cells/kg, at least or at least about or at or about 3×10⁸ cells/kg, at least or at least about or at or about 4×10⁸ cells/kg, at least or at least about or at or about 5×10⁸ cells/kg, at least or at least about or at or about 6×10⁸ cells/kg, at least or at least about or at or about 7×10⁸ cells/kg, at least or at least about or at or about 8×10⁸ cells/kg, at least or at least about or at or about 9×10⁸ cells/kg, at least or at least about or at or about 1×10⁹ cells/kg, or at least or at least about or at or about 2×10⁹ cells/kg, at least or at least about or at or about 3×10⁹ cells/kg, at least or at least about or at or about 4×10⁹ cells/kg, at least or at least about or at or about 5×10⁹ cells/kg, at least or at least about or at or about 6×10⁹ cells/kg, at least or at least about or at or about 7×10⁹ cells/kg, at least or at least about or at or about 8×10⁹ cells/kg, at least or at least about or at or about 9×10⁹ cells/kg, at least or at least about or at or about 1×10¹⁰ cells/kg, or at least or at least about or at or about 2×10¹⁰ cells/kg, at least or at least about or at or about 3×10¹⁰ cells/kg, at least or at least about or at or about 4×10¹⁰ cells/kg, at least or at least about or at or about 5×10¹⁰ cells/kg, at least or at least about or at or about 6×10¹⁰ cells/kg, at least or at least about or at or about 7×10¹⁰ cells/kg, at least or at least about or at or about 8×10¹⁰ cells/kg, at least or at least about or at or about 9×10¹⁰ cells/kg, at least or at least about or at or about 1×10¹¹ cells/kg, or at least or at least about or at or about 2×10¹¹ cells/kg, Dosages may vary depending on attributes particular to the disease or disorder and/or patient and/or other treatments. In some embodiments, the dose of cells is a flat dose of cells or fixed dose of cells such that the dose of cells is not tied to or based on the body surface area or weight of a subject.

In some embodiments, the dose of induced immune cells of the invention, is administered to the subject as a single dose or is administered only one time within a period of two weeks, one month, three months, six months, 1 year or more.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose or as a plurality of compositions, provided in multiple individual compositions or infusions, over a specified period of time, such as over no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some aspects, the cells of the dose are administered in a single pharmaceutical composition. In some embodiments, the induced immune cells of the dose are administered in a plurality of compositions, collectively containing the cells of the dose.

In some embodiments, the term "split dose" refers to a dose that is split so that it is administered over more than one day. This type of dosing is encompassed by the present methods and is considered to be a single dose.

Thus, the dose of induced immune cells may be administered as a split dose, e.g., a split dose administered over time. For example, in some embodiments, the dose may be administered to the subject over 2 days or over 3 days. Exemplary methods for split dosing include administering 25% of the dose on the first day and administering the remaining 75% of the dose on the second day. In other embodiments, 33% of the dose may be administered on the first day and the remaining 67% administered on the second day. In some aspects, 10% of the dose is administered on the first day, 30% of the dose is administered on the second day, and 60% of the dose is administered on the third day. In some embodiments, the split dose is not spread over more than 3 days.

In some embodiments, the administration of the composition or dose, e.g., administration of the plurality of cell compositions, involves administration of the cell compositions separately. In some aspects, the separate administrations are carried out simultaneously, or sequentially, in any order. In some embodiments, the dose comprises a first composition and a second composition, and the first composition and second composition are administered within 48 hours of each other, such as no more than 36 hours of each other or not more than 24 hours of each other. In some embodiments, the first composition and second composition are administered 0 to 12 hours apart, 0 to 6 hours apart or 0 to 2 hours apart. In some embodiments, the initiation of administration of the first composition and the initiation of administration of the second composition are carried out no more than 2 hours, no more than 1 hour, or no more than 30 minutes apart, no more than 15 minutes, no more than 10 minutes or no more than 5 minutes apart. In some embodiments, the initiation and/or completion of administration of the first composition and the completion and/or initiation of administration of the second composition are carried out no more than 2 hours, no more than 1 hour, or no more than 30 minutes apart, no more than 15 minutes, no more than 10 minutes or no more than 5 minutes apart.

In some embodiments, the first composition and the second composition is mixed prior to the administration into the subject. In some embodiments, the first composition and the second composition is mixed shortly (e.g., within 6 hours, 5 hours, 4 hours, 3 hours, 2 hours, 1.5 hours, 1 hour, or 0.5 hour) before the administration, In some embodiments, the first composition and the second composition is mixed immediately before the administration.

In some embodiments, the subject receives multiple doses, e.g., two or more doses or multiple consecutive doses, of the induced immune cells. In some embodiments, two doses are administered to a subject. In some embodiments, the subject receives the consecutive dose, e.g., second dose, is administered approximately 4, 5, 6, 7, 8, 9, 10, 11, 12, 13, 14, 15, 16, 17, 18, 19, 20 or 21 days after the first dose. In some embodiments, multiple consecutive doses are administered following the first dose, such that an additional dose or doses are administered following administration of the consecutive dose. In some aspects, the number of cells administered to the subject in the additional dose is the same as or similar to the first dose and/or consecutive dose. In some embodiments, the additional dose or doses are larger than prior doses.

### Preventive treatment

Another aspect of the invention relates to a pharmaceutical composition comprising immune cells induced by at least a compound of formula (I) for its use in the prevention of immune-mediated diseases. Advantageously, the immune-mediated diseases are selected from: neurodegenerative diseases, inflammatory diseases, allergic diseases, adverse events related to transplantation, autoimmune diseases, metabolic diseases with an inflammatory component, inflammation facilitating cancer development or progression, and aging.

As used herein, the term "prevention" or "prophylaxis" or "preventative treatment" or "prophylactic treatment" comprises a treatment leading to the prevention of a disease as well as a treatment reducing and/or delaying the incidence of a disease or the risk of occurrence of the disease.

In a particularly advantageous embodiment, the present invention relates to a pharmaceutical composition comprising a therapeutically efficacious quantity of immune cells induced by at least a compound of formula (I) as active ingredient, for its use in the prevention of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression, and aging.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression, and aging.

In a particularly advantageous embodiment, the present invention relates to a pharmaceutical composition comprising a therapeutically efficacious quantity of immune cells induced by at least a compound of formula (I) as active ingredient and and at least one pharmaceutically carrier as defined above, for its use in the prevention of neurodegenerative diseases. The term "neurodegenerative disease", as used herein, refers to a condition or disorder in which neuronal cells are lost due to cell death bringing about a deterioration of cognitive functions or result in damage, dysfunction, or complications that may be characterized by neurological, neurodegenerative, physiological, psychological, or behavioral aberrations. Neurodegenerative diseases include, without limitation, multiple sclerosis, autistic spectrum disorder, depression, age-related macular degeneration, Creutzfeldt-Jakob disease, Alzheimer's Disease, radiotherapy induced dementia, axon injury, acute cortical spreading depression, alpha-synucleinopathies, brain ischemia, Huntington's disease, Guillain-Barre syndrome, permanent focal cerebral ischemia, peripheral nerve regeneration, post-status epilepticus model, spinal cord injury, sporadic amyotrophic lateral sclerosis, transmissible spongiform encephalopathy, myasthenia gravis, obsessive-compulsive disorder, optic neuritis, retinal degeneration, dry eye syndrome DES, Sjogren's syndrome, chronic idiopathic demyelinating disease (CID), anxiety, compulsive disorders (such as compulsive obsessive disorders), meningitis, neuroses, psychoses, insomnia and sleeping disorder, sleep apnoea, and drug abuse.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of inflammatory diseases.

Advantageously, the inflammatory disease is selected among : Inflammatory diseases can include inflammatory diseases of the digestive apparatus, especially including the intestine (and particularly the colon in the case of irritable bowel syndrome, ulcero-haemorrhagic rectocolitis or Crohn's disease); pancreatitis, hepatitis (acute and chronic), inflammatory bladder pathologies and gastritis; inflammatory diseases of the locomotor apparatus, including rheumatoid arthritis, osteoarthritis, osteoporosis, traumatic arthritis, post-infection arthritis, muscular degeneration and dermatomyositis; inflammatory diseases of the urogenital apparatus and especially glomerulonephritis; inflammatory diseases of the cardiac apparatus and especially pericarditis and myocarditis and diseases including those for which inflammation is an underlying factor (such as atherosclerosis, transplant atherosclerosis, peripheral vascular diseases, inflammatory vascular diseases, intermittent claudication or limping, restenosis, strokes, transient ischaemic attacks, myocardial ischaemia and myocardial infarction), hypertension, hyperlipidaemia, coronary diseases, unstable angina (or angina pectoris), thrombosis, platelet aggregation induced by thrombin and/or the consequences of thrombosis and/or of the formation of atheroma plaques; inflammatory diseases of the respiratory and ORL apparatus, especially including asthma, acute respiratory distress syndrome, hayfever, allergic rhinitis and chronic obstructive pulmonary disease; inflammatory diseases of the skin, and especially urticaria, scleroderma, contact dermatitis, atopic dermatitis, psoriasis, ichthyosis, acne and other forms of folliculitis, rosacea and alopecia; the treatment of pain, irrespective of its origin, such as post-operative pain, neuromuscular pain, headaches, cancer-related pain, dental pain or osteoarticular pain; modulating pigmentation, for the treatment of: diseases with pigmentation disorders and especially benign dermatoses such as vitiligo, albinism, melasma, lentigo, ephelides, melanocytic naevus and all post-inflammatory pigmentations; and also pigmented tumours such as melanomas and their local (permeation nodules), regional or systemic metastases; photo-protection for the purpose of preventing: the harmful effects of sunlight, such as actinic erythema, cutaneous ageing, skin cancer (spinocellular, basocellular and melanoma) and especially diseases that accelerate its occurrence (xeroderma pigmentosum, basocellular naevus syndrome and familial melanoma); photodermatoses caused by exogenous photosensitizers and especially those caused by contact photosensitizers (for example furocoumarins, halogenated salicylanilides and derivatives, and local sulfamides and derivatives) or those caused by systemic photosensitizers (for example psoralenes, tetracyclines, sulfamides, phenothiazines, nalidixic acid and tricyclic antidepressants); bouts or outbreaks of dermatosis with photosensitivity and especiallylight-aggravated dermatoses (for example lupus erythematosus, recurrent herpes, congenital poikilodermal or telangiectatic conditions with photosensitivity (Bloom's syndrome, Cockayne's syndrome or Rothmund-Thomson syndrome), actinic lichen planus, actinic granuloma, superficial disseminated actinic porokeratosis, acne rosacea, juvenile acne, bullous dermatosis, Darier's disease, lymphoma cutis, psoriasis, atopic dermatitis, contact eczema, Chronic Actinic Dermatosis (CAD), follicular mucinosis, erythema multiforme, fixed drug eruption, cutaneous lymphocytoma, reticular erythematous mucinosis, and melasma);dermatoses with photosensitivity by deficiency of the protective system with anomalies of melanin formation or distribution (for example oculocutaneous albinism, phenylketonuria, hypopituitarism, vitiligo and piebaldism) and with deficiency of the DNA repair systems (for example xeroderma pigmentosum and Cockayne's syndrome), dermatoses with photosensitivity via metabolic anomalies, for instance cutaneous porphyria (for example tardive cutaneous porphyria, mixed porphyria, erythropoietic protoporphyria, congenital erythropoietic porphyria (Günther's disease), and erythropoietic coproporphyria), pellagra or pellagroid erythema (for example pellagra, pellagroid erythemas and tryptophan metabolism disorders); bouts or outbreaks of idiopathic photodermatoses and especially PMLE (polymorphic light eruption), benign summer light eruption, actinic prurigo, persistent photosensitizations (actinic reticuloid, remanent photosensitizations and photosensitive eczema), solar urticaria, hydroa vacciniforme, juvenile spring eruption and solar pruritus;modifying the colour of the skin or head hair and bodily hair, and especially by tanning the skin by increasing melanin synthesis or bleaching it by interfering with melanin synthesis, but also by preventing the bleaching or greying of head hair or bodily hair (for example canities and piebaldism); and also modifying the colour of head hair and bodily hair in cosmetic indications; modifying the sebaceous functions, and especially the treatment of: hyperseborrhoea complaints and especially acne, seborrhoeic dermatitis, greasy skin and greasy hair, hyperseborrhoea in Parkinson's disease and epilepsy and hyperandrogenism; complaints with reduction of sebaceous secretion and especially xerosis and all forms of dry skin; benign or malignant proliferation of sebocytes and the sebaceous glands; inflammatory complaints of the pilosebaceous follicles and especially acne, boils, anthrax and folliculitis; male or female sexual dysfunctions; male sexual dysfunctions including, but not limited to, impotence, loss of libido and erectile dysfunction; female sexual dysfunctions including, but not limited to, sexual stimulation disorders or desire-related disorders, sexual receptivity, orgasm, and disturbances of the major points of sexual function; pain, premature labour, dysmenorrhoea, excessive menstruation, and endometriosis; disorders related to weight but not limited to obesity and anorexia (such as modification or impairment of appetite, metabolism of the spleen, or the vocable irreproachable taking of fat or carbohydrates); diabetes mellitus (by tolerance to glucose doses and/or reduction of insulin resistance); cancer and in particular lung cancer, prostate cancer, bowel cancer, breast cancer, ovarian cancer, bone cancer or angiogenesis disorders including the formation or growth of solid tumours.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of immune-mediated disease. Immune-mediated diseases include, for example, but not limited to, asthma, allergies, arthritis (e.g., rheumatoid arthritis, psoriatic arthritis, and ankylosing spondylitis), juvenile arthritis, inflammatory bowel diseases (e.g., ulcerative colitis and Crohn's disease), endocrinopathies (e.g., type 1 diabetes and Graves' disease), neurodegenerative diseases (e.g., multiple sclerosis (MS)), depression, obsessive-compulsive disorder, optic neuritis, retinal degeneration, dry eye syndrome DES, Sjogren's syndrome, vascular diseases (e.g., autoimmune hearing loss, systemic vasculitis, and atherosclerosis), and skin diseases (e.g., acne vulgaris dermatomyositis, pemphigus, systemic lupus erythematosus (SLE), discoid lupus erthematosus, scleroderma, psoriasis, plaque psoriasis, vasculitics, vitiligo and alopecias). Hashimoto's thyroiditis, pernicious anemia, Cushing's disease, Addison's disease, chronic active hepatitis, polycystic ovary syndrome (PCOS), celiac disease, pemphigus, transplant rejection (allograft transplant rejection), graft-versus-host disease (GVDH).

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of allergic diseases. As used herein, the term "allergy" or "allergies" or "allergic reaction" or "allergic response" refers to a disorder (or improper reaction) of the immune system. Allergic reactions occur to normally harmless environmental substances known as allergens; these reactions are acquired, predictable, and rapid. Strictly, allergy is one of four forms of hypersensitivity and is called type I (or immediate) hypersensitivity. It is characterized by excessive activation of certain white blood cells called mast cells and basophils by a type of antibody known as IgE, resulting in an extreme inflammatory response. Common allergic reactions include eczema, hives, hay fever, asthma, food allergies, and reactions to the venom of stinging insects such as wasps and bees. Mild allergies like hay fever are highly prevalent in the human population and cause symptoms such as allergic conjunctivitis, itchiness, and runny nose. Allergies can play a major role in conditions such as asthma. In some people, severe allergies to environmental or dietary allergens or to medication may result in life-threatening anaphylactic reactions and potentially death. The treatment of allergies via immune suppression is therefore one embodiment of the present invention.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of adverse events related to transplantation.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of autoimmune diseases.

Examples of autoimmune disease include, but are not limited to arthritis (rheumatoid arthritis, juvenile-onset rheumatoid arthritis, osteoarthritis, psoriatic arthritis, and ankylosing spondylitis), psoriasis, dermatitis including atopic dermatitis, chronic idiopathic urticaria, including chronic autoimmune urticaria, polymyositis/ dermatomyositis, toxic epidermal necrolysis, scleroderma (including systemic scleroderma), sclerosis such as progressive systemic sclerosis, inflammatory bowel disease (IBD) (for example, Crohn's disease, ulcerative colitis, autoimmune inflammatory bowel disease), pyoderma gangrenosum, erythema nodosum, primary sclerosing cholangitis, episcleritis), respiratory distress syndrome, including adult respiratory distress syndrome (ARDS), meningitis, IgE-mediated diseases such as anaphylaxis and allergic and atopic rhinitis, encephalitis such as Rasmussen's encephalitis, uveitis or autoimmune uveitis, colitis such as microscopic colitis and collagenous colitis, glomerulonephritis (GN) such as membranous GN (membranous nephropathy), idiopathic membranous GN, membranous proliferative GN (MPGN), including Type I and Type II, and rapidly progressive GN, allergic conditions, allergic reaction, eczema, asthma, conditions involving infiltration of T cells and chronic inflammatory responses, atherosclerosis, autoimmune myocarditis, leukocyte adhesion deficiency, systemic lupus erythematosus (SLE) such as cutaneous SLE, subacute cutaneous lupus erythematosus, lupus (including nephritis, cerebritis, pediatric, non-renal, discoid, alopecia), juvenile onset (Type I) diabetes mellitus, including pediatric insulin-dependent diabetes mellitus (IDDM), adult onset diabetes mellitus (Type II diabetes), multiple sclerosis (MS) such as spino-optical MS, immune responses associated with acute and delayed hypersensitivity mediated by cytokines and T-lymphocytes, tuberculosis, sarcoidosis, granulomatosis including lymphomatoid granulomatosis, Wegener's granulomatosis, agranulocytosis, vasculitis (including large vessel vasculitis (including polymyalgia rheumatica and giant cell (Takayasu's) arteritis), medium vessel vasculitis (including Kawasaki's disease and polyarteritis nodosa), CNS vasculitis, systemic necrotizing vasculitis, and ANCA-associated vasculitis, such as Churg-Strauss vasculitis or syndrome (CSS)), temporal arteritis, aplastic anemia, Coombs positive anemia, Diamond Blackfan anemia, hemolytic anemia or immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, CNS inflammatory disorders, multiple organ injury syndrome, antigen-antibody complex mediated diseases, anti-glomerular basement membrane disease, antiphospholipid antibody syndrome, allergic neuritis, Bechet's or Behcet's disease, Castleman's syndrome, Goodpasture's syndrome, Reynaud's syndrome, Sjogren's syndrome, Stevens-Johnson syndrome, pemphigoid such as pemphigoid bullous, pemphigus (including vulgaris, foliaceus, and pemphigus mucus-membrane pemphigoid), autoimmune polyendocrinopathies, Reiter's disease, immune complex nephritis, chronic neuropathy such as IgM polyneuropathies or IgM-mediated neuropathy, thrombocytopenia (as developed by myocardial infarction patients, for example), including thrombotic thrombocytopenic purpura (TTP) and autoimmune or immune-mediated thrombocytopenia such as idiopathic thrombocytopenic purpura (ITP) including chronic or acute ITP, autoimmune disease of the testis and ovary including autoimmune orchitis and oophoritis, primary hypothyroidism, hypoparathyroidism, autoimmune endocrine diseases including thyroiditis such as autoimmune thyroiditis, chronic thyroiditis (Hashimoto's thyroiditis), or subacute thyroiditis, autoimmune thyroid disease, idiopathic hypothyroidism, Addison's disease, Grave's disease, polyglandular syndromes such as autoimmune polyglandular syndromes (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis, myasthenia gravis, cerebellar degeneration, limbic and/or brainstem encephalitis, neuromyotonia, opsoclonus or opsoclonus myoclonus syndrome (OMS), and sensory neuropathy, Sheehan's syndrome, autoimmune hepatitis, chronic hepatitis, lupoid hepatitis, chronic active hepatitis or autoimmune chronic active hepatitis, lymphoid interstitial pneumonitis, bronchiolitis obliterans (non-transplant) vs NSIP, Guillain Barre syndrome, Berger's disease (IgA nephropathy), primary biliary cirrhosis, celiac sprue (gluten enteropathy), refractory sprue, dermatitis herpetiformis, cryoglobulinemia, amyotrophic lateral sclerosis (ALS; Lou Gehrig's disease), coronary artery disease, autoimmune inner ear disease (AIED) or autoimmune hearing loss, opsoclonus myoclonus syndrome (OMS), polychondritis such as refractory polychondritis, pulmonary alveolar proteinosis, amyloidosis, giant cell hepatitis, scleritis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal segmental glomerulosclerosis (FSGS), endocrine ophthalmopathy, uveoretinitis, autoimmune hepatological disorder, fibromyalgia, multiple endocrine failure, Schmidt's syndrome, adrenalitis, gastric atrophy, presenile dementia, demyelinating diseases, Dressler's syndrome, alopecia areata, CREST syndrome (calcinosis, Raynaud's phenomenon, esophageal dysmotility, sclerodactyly, and telangiectasia), male and female autoimmune infertility, ankylosing spondolytis, mixed connective tissue disease, Chagas' disease, rheumatic fever, recurrent abortion, farmer's lung, erythema multiforme, post-cardiotomy syndrome, Cushing's syndrome, bird-fancier's lung, Alport's syndrome, alveolitis such as allergic alveolitis and fibrosing alveolitis, interstitial lung disease, transfusion reaction, leprosy, malaria, leishmaniasis, kypanosomiasis, schistosomiasis, ascariasis, aspergillosis, Sampter's syndrome, Caplan's syndrome, dengue, endocarditis, endomyocardial fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, or Fuch's cyclitis, Henoch-Schonlein purpura, human immunodeficiency virus (HIV) infection, echovirus infection, cardiomyopathy, Alzheimer's disease, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, and giant cell polymyalgia. The treatment of autoimmune disorders via immune suppression is therefore one embodiment of the present invention.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of metabolic diseases with an inflammatory component such as arteriosclerosis with resulting coronary heart diseases, adiposity or diabetes.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of aging.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the prevention of inflammation facilitating cancer development or progression

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above and a second active ingredient, for its use in the prevention of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression, and aging.

Another aspect of the invention relates to a method for the preventive treatment of immune-mediated diseases. Advantageously, the immune-mediated disease is selected from : neurodegenerative diseases, inflammatory diseases, allergic diseases, adverse events related to transplantation, autoimmune diseases, metabolic diseases with an inflammatory component, inflammation facilitating cancer development or progression and aging, comprising administration to the patient of immune cells induced by at least a compound of formula (I) or of a pharmaceutical composition comprising a therapeutically efficacious quantity of immune cells induced by at least a compound of formula (I) as active ingredient and and at least one pharmaceutically acceptable carrier as defined above as defined above.

According to the present invention, the immune cells induced by at least a compound of formula (I) are particularly efficacious for preventing such diseases in adoptive cell therapy, since the immune cells induced by at least a compound of formula (I) act directly in the target organ, where they rapidly home and locally intercept the activation of microglia and CNS-associated macrophages. The immune cells induced by at least a compound of formula (I) directly intercept the inflammatory reaction in the target organ. The immune cells induced by at least a compound of formula (I) directly produce IL-10 at inflamed sites.

In a particularly advantageous embodiment, the present invention relates to a method for the preventive treatment of immune-mediated diseases. Advantageously, the immune-mediated disease is selected from : neurodegenerative diseases, inflammatory diseases, allergic diseases, adverse events related to transplantation, autoimmune diseases, metabolic diseases with an inflammatory component, inflammation facilitating cancer development or progression and aging, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the preventive treatment of neurodegenerative diseases, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the preventive treatment of inflammatory diseases, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the preventive treatment of immune-mediated diseases, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the preventive treatment of allergic diseases, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the preventive treatment of adverse events related to transplantation, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the preventive treatment of metabolic diseases with an inflammatory component, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the preventive treatment of aging, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the preventive treatment of inflammation facilitating cancer development or progression, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression, and aging.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of neurodegenerative diseases.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of inflammatory diseases.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of immune-mediated disease.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of allergic diseases.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of adverse events related to transplantation.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of autoimmune diseases.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of metabolic diseases with an inflammatory component.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of aging.

In a particularly advantageous embodiment, the present invention relates to the use of a pharmaceutical composition comprising a therapeutically efficacious quantity of immune cells induced by at least a compound of formula (I) as active ingredient in the production of a medicament intended for the prevention of inflammation facilitating cancer development or progression

### Curative Treatment

Another aspect of the invention relates to a pharmaceutical composition comprising immune cells induced by at least a compound of formula (I) for its use in the treatment of immune-mediated diseases. Advantageously, the immune-mediated diseases are selected from: neurodegenerative diseases, inflammatory diseases, allergic diseases, adverse events related to transplantation, autoimmune diseases, metabolic diseases with an inflammatory component, inflammation facilitating cancer development or progression, and aging.

As used herein, the term "treatment" or "curative treatment" is defined as a treatment leading to recovery or treatment that relieves, ameliorates and/or eliminates, reduces and/or stabilizes the symptoms of a disease or the suffering that it elicits.

In a particularly advantageous embodiment, the present invention relates to a pharmaceutical composition comprising a therapeutically efficacious quantity of immune cells induced by at least a compound of formula (I) as active ingredient, for its use in the treatment of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression, and aging.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression, and aging.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of neurodegenerative diseases.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of inflammatory diseases.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of immune-mediated disease.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of allergic diseases.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of adverse events related to transplantation.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of autoimmune diseases.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of metabolic diseases with an inflammatory component.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of aging.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above, for its use in the treatment of inflammation facilitating cancer development or progression.

In a particularly advantageous embodiment, the present invention relates to a pharmaceutical composition comprising a therapeutically efficacious quantity of immune cells induced by at least a compound of formula (I) as active ingredient and and at least one pharmaceutically acceptable carrier as defined above and a second active ingredient, for its use in the treatment of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression, and aging.

Another aspect of the invention relates to a method for the curative treatment of immune-mediated diseases. Advantageously, the immune-mediated disease is selected from : neurodegenerative diseases, inflammatory diseases, allergic diseases, adverse events related to transplantation, autoimmune diseases, metabolic diseases with an inflammatory component, inflammation facilitating cancer development or progression and aging, comprising administration to the patient of immune cells induced by at least a compound of formula (I) or of a pharmaceutical composition comprising a therapeutically efficacious quantity of immune cells induced by at least a compound of formula (I) as active ingredient and and at least one pharmaceutically acceptable carrier as defined above.

In a particularly advantageous embodiment, the present invention relates to a method for the curative treatment of immune-mediated diseases. Advantageously, the immune-mediated disease is selected from : neurodegenerative diseases, inflammatory diseases, allergic diseases, adverse events related to transplantation, autoimmune diseases, metabolic diseases with an inflammatory component, inflammation facilitating cancer development or progression and aging, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above. In a particularly advantageous embodiment, the present invention relates to a method for the curative treatment of neurodegenerative diseases, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the curative treatment of inflammatory diseases, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the curative treatment of immune-mediated diseases, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the curative treatment of allergic diseases, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the curative treatment of adverse events related to transplantation, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the curative treatment of metabolic diseases with an inflammatory component, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the curative treatment of aging, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

In a particularly advantageous embodiment, the present invention relates to a method for the curative treatment of inflammation facilitating cancer development or progression, comprising administration to the patient of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

Another aspect of the invention relates to the use of a pharmaceutical composition comprising a therapeutically efficacious quantity of immune cells induced by at least a compound of formula (I) as active ingredient in the production of a medicament intended for the treatment of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression and aging.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of neurodegenerative diseases.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of inflammatory diseases.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of immune-mediated disease.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of allergic diseases.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of adverse events related to transplantation.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of autoimmune diseases.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of metabolic diseases with an inflammatory component.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of aging.

In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of inflammation facilitating cancer development or progression.

Another subject of the invention relates to a method for the curative treatment of immune-mediated diseases. Advantageously, the immune-mediated disease is selected from : neurodegenerative diseases, inflammatory diseases, allergic diseases, adverse events related to transplantation, autoimmune diseases, metabolic diseases with an inflammatory component, inflammation facilitating cancer development or progression, and aging, in patients requiring an administration, comprising administration to said patients of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above.

Another subject of the invention relates to a method for the curative treatment of immune-mediated diseases. Advantageously, the immune-mediated disease is selected from : neurodegenerative diseases, inflammatory diseases, allergic diseases, adverse events related to transplantation, autoimmune diseases, metabolic diseases with an inflammatory component, inflammation facilitating cancer development or progression, and aging in patients requiring an administration, comprising administration to said patients of immune cells induced by at least a compound of formula (I) or of a pharmaceutical composition comprising a therapeutically efficacious quantity of immune cells induced by at least a compound of formula (I) as active ingredient and at least one pharmaceutically acceptable carrier as defined above and a second active ingredient as defined above. In a particularly advantageous embodiment, the present invention relates to the use of a pharmaceutical composition comprising a therapeutically efficacious quantity of immune cells induced by at least a compound of formula (I) as active ingredient in the production of a medicament intended for the treatment of immune-mediated diseases. Advantageously, the immune-mediated disease is selected from: neurodegenerative diseases, inflammatory diseases, allergic diseases, adverse events related to transplantation, autoimmune diseases, metabolic diseases with an inflammatory component, inflammation facilitating cancer development or progression, and aging.

Another aspect of the invention provides a compound of formula (I): 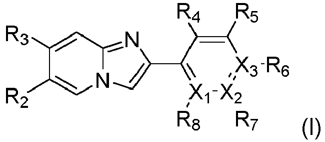 wherein:
- X₁, X₂, X₃ identical or different, are each independently selected from a nitrogen atom and a carbon atom ;
- R₂ and R₃, identical or different, are each independently selected from:
   - a hydrogen atom,
   - a halogen atom,
   - a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
   - a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a -NO₂ group, a -OCF₃ group, a -CF₃ group;
   - a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
   - a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a - NO₂ group, a -OCF₃ group, a -CF₃ group;
- R₄, R₅, R₆, R₇ and R₈, identical or different, are each independently selected from :
   - a hydrogen atom,
   - a cyano group,
   - a halogen atom, and
   - a -NHCOR_{b} group, wherein R_{b} is selected from a (C₁-C₆)cycloalkyl group, a (C₁-C₄)alkyl group, a (C₁-C₄)allyl group, a (C₁-C₄)alkoxy group, a pyridine group and a phenyl group substituted by (Z₈)_{b}, wherein : b is selected from 0, 1, 2, 3, 4 and 5, and Z₈, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
      with the proviso that
   - if X₁ is a nitrogen atom, R₈ is absent,
   - if X₂ is a nitrogen atom, R₇ is absent,
   - if X₃ is a nitrogen atom, R₆ is absent,
      and with the provisio that the compound of formula (I) is not one of the following compounds 6-iodo-2-phenylimidazo[1,2-a]pyridine, 6-iodo-2-(pyridin-3-yl)imidazo[1,2-a]pyridine, N-(2-(4-fluorophenyl)imidazo[1,2-a]pyridin-6-yl)benzamide, . N-(2-(4-fluorophenyl)imidazo[1,2-a]pyridin-6-yl)-2-methoxybenzamide, 2-(3,4-difluorophenyl)-6-iodoimidazo[1,2-a]pyridine, 2-(4-fluorophenyl)-6-iodoimidazo[1,2-a]pyridine and 2-(3-fluorophenyl)-6-iodoimidazo[1,2-a]pyridine.

### EXEMPLES

### Example 1 : Synthesis of the compound of formula (I)

The examples that follow illustrate the present invention without limiting the scope thereof. The following examples illustrate in detail the preparation of some compounds according to the invention. The structures of the products obtained have been confirmed by NMR analyses and mass spectroscopy.

### Synthesis of compound 218

According to route (A), 2-amino-5-iodopyridine (500 mg, 2.27 mmol, 1eq) is placed in acetone (10 mL). 2-bromo-1-(2-fluorophenyl)ethan-1-one (493.24 mg, 2.27 mmol, 1 eq) is then added. The reaction is heated at 60°C and stirred during 20 hours. Then the formed precipitate is filtrated, washed by acetone and dried under vacuum to give 2-(2-fluorophenyl)-6-iodoimidazo[1,2-a]pyridine (218) (764 mg, 99%).

### Synthesis of compound 91

According to route (A), 2-amino-5-iodopyridine (6.82 g, 30.99 mmol, 1.24 eq) is placed in ethanol (50 mL). 2-bromo-1-(pyridin-3-yl)ethan-1-one hydrobromide (5 g, 25 mmol, 1 eq) is then added in presence of NaHCO₃ (3.045 g, 36.25 mmol, 1.45 eq). The reaction mixture is heated at 80°C and stirred during 16 hours. The reaction is concentrated under reduce pressure and the resulting residue is purified by column chromatography on silica gel to give 6-iodo-2-(pyridin-3-yl)imidazo[1,2-a]pyridine (91) (642 mg, 8%).

### Synthesis of compound 133

According to route (B), 6-iodo-2-(pyridin-3-yl)imidazo[1,2-a]pyridine (91) (50 mg, 0.16 mmol, 1 eq) is placed in toluene (1 mL). 2-methoxybenzamide (1.2 eq., 28.33 mg, 0.19 mmol is then added in presence of K₃PO₄ (66.305 mg, 0.31 mmol, 2 eq), copper(I) iodide (5.95 mg, 0.031 mmol, 0.2 eq), and trans-N,N'-dimethylcyclohexane-1,2-diamine (4.44 mg, 0.031 mmol, 0.2 eq). The reaction mixture is heated at 120°C and stirred during 48 hours in sealed tube under argon. The reaction mixture is diluted with dichloromethane and filtrated through a celite pad. The filtrate is concentrated under reduced pressure. The resulting residue is purified by column chromatography on silica gel to give 2-methoxy-N-(2-(pyridin-3-yl)imidazo[1,2-a]pyridin-6-yl)benzamide (133) (10 mg, 19%).

### Example 2: Identification of compound of the invention with a comparable IL-10 inducing capacity in human B cells.

Human B cells isolated magnetically through negative selection (B Cell Isolation Kit II, human, Miltenyi ref: 130-091-151) were cultivated for 2 consecutive days in the presence of anti-IgM plus CpG in the presence of the indicated molecule (see table 2) at 0.25µM final concentration. IL-10 was measured in cell culture supernatants.

**Table 2: Tested Molecules**

| **N°** | **Structure** | **II-10 Induction Mean (pg/mL) human** |
|---|---|---|
| **133** | 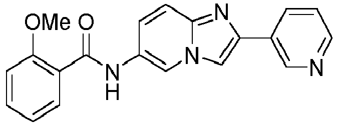 | 2,953 |
| **161** | 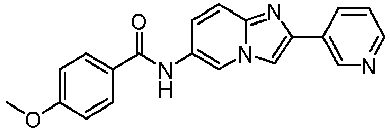 | 1,35 |
| **77** | 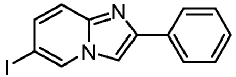 | 1,62 |
| **91** | 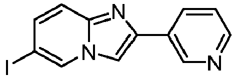 | 1,28 |

### Example 3: Effect of compounds 133, 161, 77 and 91 on IL-10 induction by human B cells

Human B cells isolated magnetically through negative selection (B Cell Isolation Kit II, human, Miltenyi ref: 130-091-151) were cultivated for 2 consecutive days in the presence of anti-IgM plus CpG in the presence of DMSO (as control) or in the presence of the indicated molecule (as identified in the attached excel file) at 0.25 microM final concentration. IL-10 was measured in cell culture supernatants. NS: non stimulated B cell culture condition.

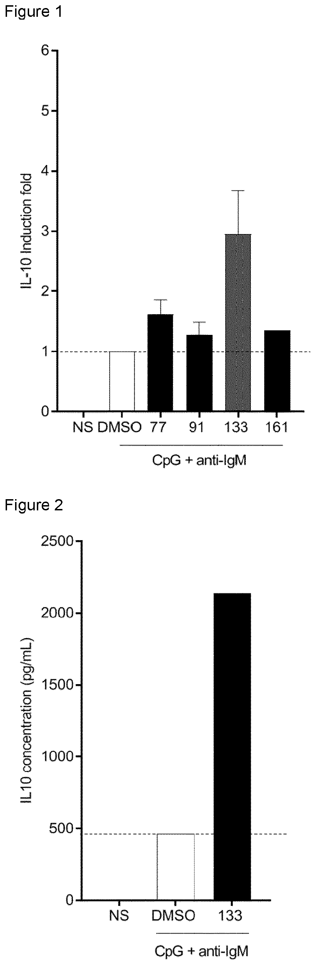

Results are presented at Figure 1.

Conclusion: These compounds increase IL-10 induction by human B cells.

### Example 4: Effect of induction of human B cells with compounds 133, 161, 77 and 91 on IL-10 production by human B cells

Human B cells isolated magnetically through negative selection (B Cell Isolation Kit II, human, Miltenyi ref: 130-091-151) were cultivated for 2 consecutive days in the presence of anti-IgM plus CpG in the presence of DMSO (as control) or in the presence of the indicated molecule (as identified in the attached excel file) at 0.25 microM final concentration. IL-10 was measured in cell culture supernatants. NS: non stimulated B cell culture condition.

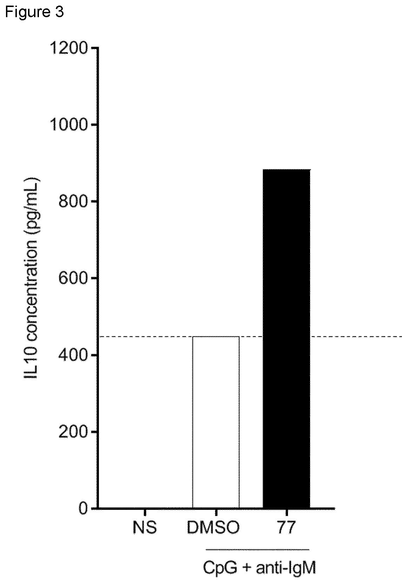

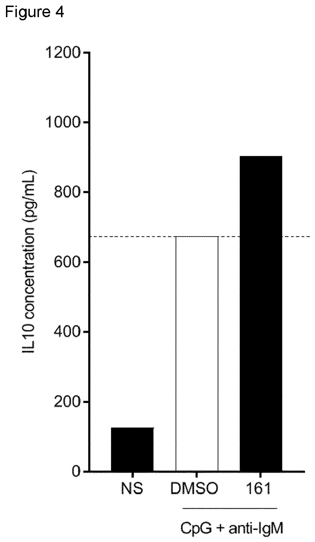

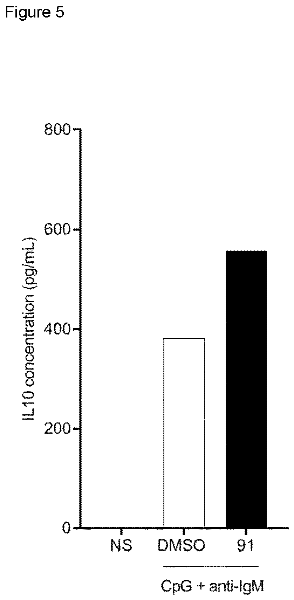

Results are presented at Figures 2 to 5.

Conclusion: These compounds increase IL-10 production by human B cells.

## Claims

1. A compound inducing production of proteins, in particular interleukin-10 (IL-10), by immune cells of formula (I): 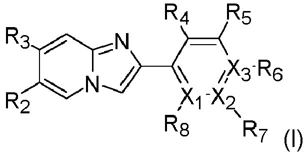 wherein:
- X₁, X₂, X₃ identical or different, are each independently selected from a nitrogen atom and a carbon atom ;
- R₂ and R₃, identical or different, are each independently selected from:
• a hydrogen atom,
• a halogen atom,
• a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a - CF₃ group ;
• a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a -NO₂ group, a -OCF₃ group, a -CF₃ group;
• a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a - CF₃ group;
• a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a -NO₂ group, a -OCF₃ group, a - CF₃ group;
- R₄, R₅, R₆, R₇ and R₈, identical or different, are each independently selected from:
• a hydrogen atom,
• a cyano group,
• a halogen atom, and
• a -NHCOR_{b} group, wherein R_{b} is selected from a (C₁-C₆)cycloalkyl group, a (C₁-C₄)alkyl group, a (C₁-C₄)allyl group, a (C₁-C₄)alkoxy group, a pyridine group and a phenyl group substituted by (Z₈)_{b}, wherein : b is selected from 0, 1, 2, 3, 4 and 5, and Z₈, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
with the proviso that
• If X₁ is a nitrogen atom, R₈ is absent,
• If X₂ is a nitrogen atom, R₇ is absent,
• If X₃ is a nitrogen atom, R₆ is absent.

2. A compound inducing production of proteins, in particular interleukin-10 (IL-10), according to claim 1, wherein :
- X₁, X₂ and X₃ are a carbon atom,
- R₂ and R₃ identical or different, are each independently selected from :
• a hydrogen atom,
• a halogen atom,
• a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z4, identical or different, are each independently selected from a halogen atom, a hydroxyl group, a (C₁-C₆) alkoxy group, a (C₁-C₄)alkyl group, a-NH2 group, a -NO2 group, a -OCF3 group, a-CF3 group,
• a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a - NO₂ group, a -OCF₃ group, a -CF₃ group;
• a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
• a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, and a-CF₃ group;
- R₄, R₅, R₆ and R₇, identical or different, are each independently selected from a hydrogen atom, a cyano group and a halogen atom.

3. A compound inducing production of proteins, in particular interleukin-10 (IL-10), according to claim 1, wherein :
- X₁ is a nitrogen atom
- X₂, X₃, are a carbon atom,
- R₂ and R₃ identical or different, are each independently selected from :
▪ a hydrogen atom,
▪ a halogen atom,
▪ a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
▪ a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a -NO₂ group, a -OCF₃ group, a -CF₃ group;
▪ a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
▪ a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a -NO₂ group, a-OCF₃ group, a -CF₃ group;
- R₄, R₅, R₆ and R₈, identical or different, are each independently selected from a hydrogen atom, a cyano group and a halogen atom.

4. A compound inducing production of proteins, in particular interleukin-10 (IL-10), according to claim 1, wherein :
- X₂ is a nitrogen atom
- X₁, X₃, are a carbon atom,
- R₂ and R₃ identical or different, are each independently selected from :
▪ a hydrogen atom,
▪ a halogen atom,
▪ a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
▪ a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a -NO₂ group, a -OCF₃ group, a -CF₃ group;
▪ a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
▪ a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a -NO₂ group, a-OCF₃ group, a -CF₃ group;
- R₄, R₅, R₆ and R₈, identical or different, are each independently selected from a hydrogen atom, a cyano group and a halogen atom.

5. A compound inducing production of proteins, in particular interleukin-10 (IL-10), according to claim 1, wherein :
- X₃ is a nitrogen atom
- X₁ and X₂ are a carbon atom,
- R₂ and R₃ identical or different, are each independently selected from :
▪ a hydrogen atom,
▪ a halogen atom,
▪ a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z4, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
▪ a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a -NO₂ group, a -OCF₃ group, a -CF₃ group;
▪ a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
▪ a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
- R₄, R₅, R₇ and R₈, identical or different, are each independently selected from a hydrogen atom, a cyano group and a halogen atom.

6. A compound inducing production of proteins, in particular interleukin-10 (IL-10), according to claim 1, wherein :
- X₂ is a nitrogen atom,
- X₁ and X₃ are a carbon atom,
- R₂ and R₃ identical or different, are each independently selected from a hydrogen atom and a halogen atom, and a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group;
- R₄, R₅, R₆ and R₈, identical or different, are each independently selected from a hydrogen atom, a cyano group and a halogen atom.

7. A compound inducing production of proteins, in particular interleukin-10 (IL-10), according to claim 1, wherein :
- X₂ is a nitrogen atom or a carbon atom,
- X₁ and X₃ are a carbon atom,
- R₂ and R₃ identical or different, are each independently selected from a hydrogen atom and a halogen atom,
- R₄, R₅, R₆ and R₈, identical or different, are each independently selected from a hydrogen atom, a cyano group and a halogen atom.

8. A compound inducing production of proteins, in particular interleukin-10 (IL-10) according to any one of claims 1 to 7, selected from

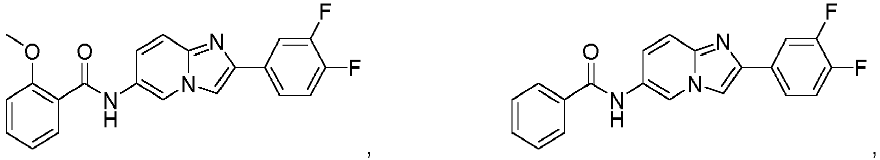

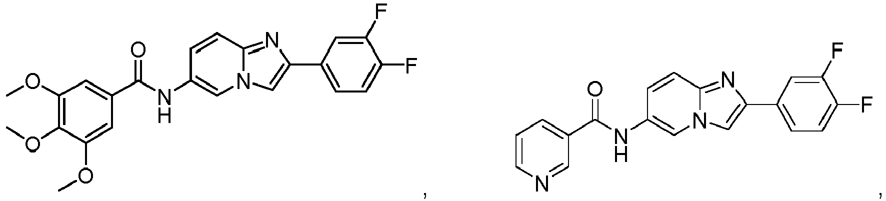

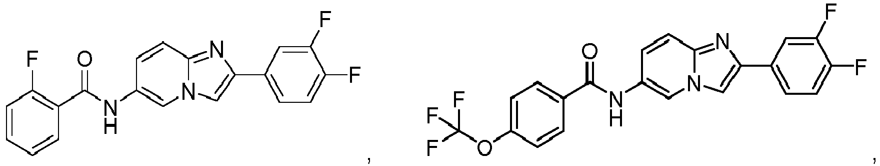

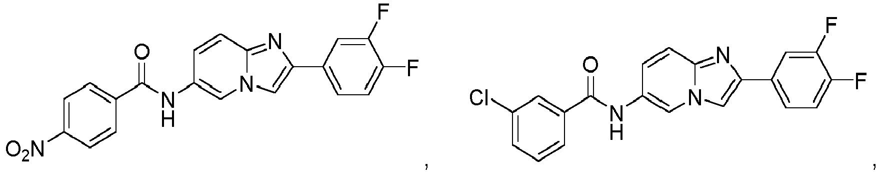

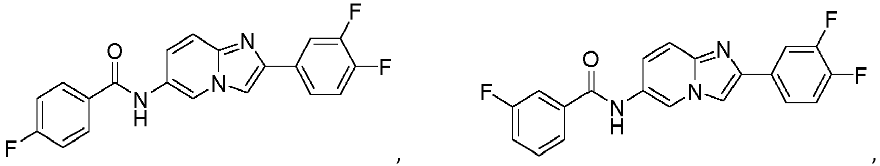

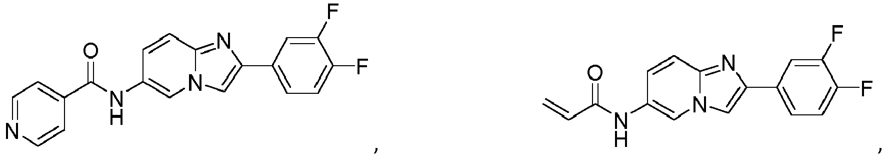

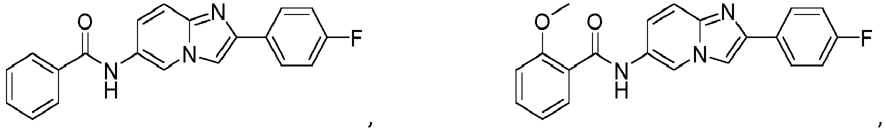

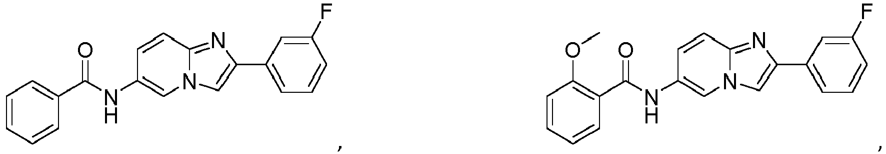

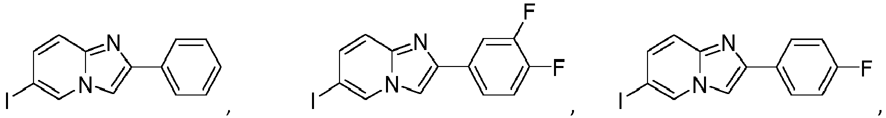

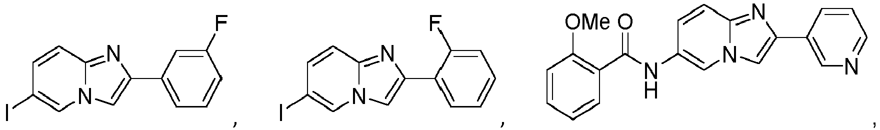

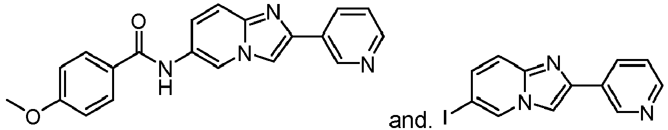

9. A compound inducing production of proteins, in particular interleukin-10 (IL-10) according to any one of claims 1 to 8, selected from:

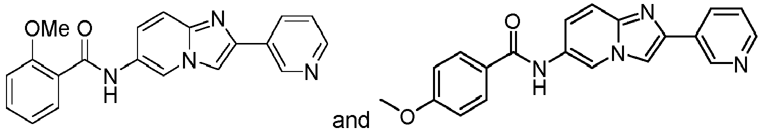

10. A compound inducing production of proteins, in particular interleukin-10 (IL-10) according to any one of claims 1 to 8, selected from:

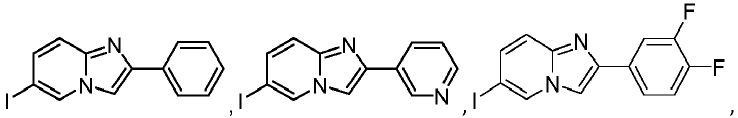

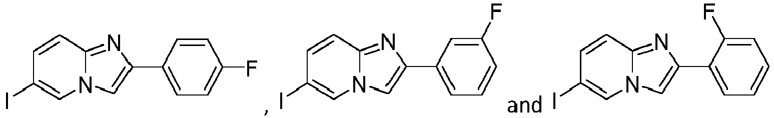

11. A compound inducing production of proteins, in particular interleukin-10 (IL-10) according to any one of claims 1 to 10, wherein the immune cells are selected among T lymphocytes, B lymphocytes, dendritic cells, natural killer cells, innate lymphoid cells, mesenchymal cells and myeloid cells.

12. Method for inducing an immune cell **characterized in that** said immune cells are contacting with at least one compound inducing production of proteins, in particular interleukin-10 according to any one of claims 1 to 11.

13. Immune cell capable of producing proteins, in particular interleukin 10 obtained by the method of claim 12.

14. Pharmaceutical composition comprising at least a dose of induced immune cells according to claim 13, and at least one pharmaceutically carrier.

15. Pharmaceutical composition according to claim 14, wherein the pharmaceutical composition further comprises at least one second active ingredient.

16. Pharmaceutical composition according to any one of claims 14 to 15 for its use in the prevention and/or treatment of immune-mediated diseases.

17. Pharmaceutical composition according to claim 16, wherein the immune-mediated diseases are selected from neurodegenerative diseases, inflammatory diseases, allergic diseases, adverse events related to transplantation, autoimmune diseases, metabolic diseases with an inflammatory component, inflammation facilitating cancer development or progression and aging.

18. A compound of formula (I): 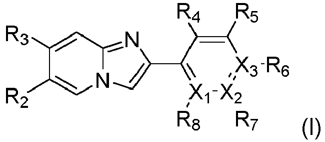 wherein:
- X₁, X₂, X₃ identical or different, are each independently selected from a nitrogen atom and a carbon atom ;
- R₂ and R₃, identical or different, are each independently selected from:
• a hydrogen atom,
• a halogen atom,
• a -NHCORₐ group, wherein Rₐ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₄)_{q}, wherein : q is selected from 0, 1, 2, 3, 4 and 5, and Z₄, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a - CF₃ group,
• a -NHR_{c} group, wherein R_{c} is selected from : a (C₁-C₆)cycloalkyl group, a (C₁-C₄)alkyl group, a (C₁-C₄)allyl group, a (C₁-C₄)alkoxy group, a pyridine group and a phenyl group substituted by (Z₅)ₚ, wherein : p is selected from 0, 1, 2, 3, 4 and 5, and Z₅, identical or different, are each independently selected from a halogen atom, a hydroxyl group, a (C₁-C₆) alkoxy group, a (C₁-C₄) alkyl group, a -NH₂ group, a -NO₂ group, a -OCF₃ group, a -CF₃ group;
• a -NHCONHR_{d} group, wherein R_{d} is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₆)ₙ, wherein : n is selected from 0, 1, 2, 3, 4 and 5, and Z₆, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a - CF₃ group;
• a -CONHRₑ group, wherein Rₑ is selected from : a hydrogen atom, a (C₁-C₆)cycloalkyl group, a (C₁-C₄)alkyl group, a (C₁-C₄)allyl group, a (C₁-C₄)alkoxy group, a pyridine group and a phenyl group substituted by (Z₇)ⱼ, wherein : j is selected from 0, 1, 2, 3, 4 and 5, and Z₇, identical or different, are each independently selected from a halogen atom, a hydroxyl group, a C₁-C₆ alkoxy group, a (C₁-C₄)alkyl group, a -NH₂ group, a -NO₂ group, a -OCF₃ group, a - CF₃ group;
- R₄, R₅, R₆, R₇ and R₈, identical or different, are each independently selected from :
• a hydrogen atom,
• a cyano group,
• a halogen atom, and
• a -NHCOR_{b} group, wherein R_{b} is selected from a (C₁-C₆)cycloalkyl group, a (C₁-C₄)alkyl group, a (C₁-C₄)allyl group, a (C₁-C₄)alkoxy group, a pyridine group and a phenyl group substituted by (Z₈)_{b}, wherein : b is selected from 0, 1, 2, 3, 4 and 5, and Z₈, identical or different, are each independently selected from a halogen atom, a hydroxyl group, a (C₁-C₆) alkoxy group, a (C₁-C₄)alkyl group, a -NH₂ group, a -NO₂ group, a -OCF₃ group, a -CF₃ group,
with the proviso that
• if X₁ is a nitrogen atom, R₈ is absent,
• if X₂ is a nitrogen atom, R₇ is absent,
• if X₃ is a nitrogen atom, R₆ is absent,
and with the provisio that the compound of formula (I) is not one of the following compounds : 6-iodo-2-phenylimidazo[1,2-a]pyridine, 6-iodo-2-(pyridin-3-yl)imidazo[1,2-a]pyridine, N-(2-(4-fluorophenyl)imidazo[1,2-a]pyridin-6-yl)benzamide, N-(2-(4-fluorophenyl)imidazo[1,2-a]pyridin-6-yl)-2-methoxybenzamide, 2-(3,4-difluorophenyl)-6-iodoimidazo[1,2-a]pyridine, 2-(4-fluorophenyl)-6-iodoimidazo[1,2-a]pyridine and 2-(3-fluorophenyl)-6-iodoimidazo[1,2-a]pyridine.
